(19) Europäisches Patentamt
European Patent Office
Office européen des brevets

(11) **EP 4 022 284 B1**

(12) **EUROPEAN PATENT SPECIFICATION**

(45) Date of publication and mention
of the grant of the patent:
**03.06.2026 Bulletin 2026/23**

(21) Application number: **20857878.1**

(22) Date of filing: **31.08.2020**

(51) International Patent Classification (IPC):
*G01N 21/27* $^{(2006.01)}$   *G06T 7/00* $^{(2017.01)}$

(52) Cooperative Patent Classification (CPC):
**G01N 33/57585; G01N 33/545; G06N 3/045;
G06N 3/0499; G06N 3/08; G06N 3/09;
G06T 7/0012;** G01N 21/253; G06T 2207/10024;
G06T 2207/20081; G06T 2207/20084;
G06T 2207/20132; G06T 2207/30072

(86) International application number:
**PCT/US2020/048826**

(87) International publication number:
**WO 2021/042063 (04.03.2021 Gazette 2021/09)**

(54) **DETECTABLE ARRAYS FOR DISTINGUISHING ANALYTES AND DIAGNOSIS, AND METHODS AND SYSTEMS RELATED THERETO**

DETEKTIERBARE ARRAYS ZUM UNTERSCHEIDEN VON ANALYTEN UND DIAGNOSE, UND VERFAHREN UND SYSTEME IN ZUSAMMENHANG DAMIT

RÉSEAUX DÉTECTABLES POUR DISTINGUER DES ANALYTES ET DIAGNOSTIC, ET PROCÉDÉS ET SYSTÈMES ASSOCIÉS À CEUX-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.08.2019 US 201962893703 P**

(43) Date of publication of application:
**06.07.2022 Bulletin 2022/27**

(73) Proprietor: **Entopsis, Inc.
Medley, FL 33166 (US)**

(72) Inventors:
• **LIM, Francis Buan Hong**
Medley, Florida 33166 (US)
• **LEI, Tingjun**
Medley, Florida 33166 (US)
• **PILOTO, Obdulio**
Medley, Florida 33166 (US)
• **CHEONG, Ian Shen-Yi**
Medley, Florida 33166 (US)

(74) Representative: **Witek, Rafal
WTS Patent Attorneys
Witek, Sniezko & Partners
ul. Rudolfa Weigla 12
53-114 Wroclaw (PL)**

(56) References cited:
CA-A1- 2 923 228       US-A- 4 749 647
US-A1- 2006 286 570    US-A1- 2009 005 263
US-A1- 2017 175 169    US-B2- 8 652 778

• LUIS FERM◆N CAPIT◆N-VALLVEY ET AL:
"Recent developments in computer vision-based
analytical chemistry: A tutorial review",
ANALYTICA CHIMICA ACTA, vol. 899, 1 October
2015 (2015-10-01), AMSTERDAM, NL, pages 23 -
56, XP055400184, ISSN: 0003-2670, DOI: 10.1016/
j.aca.2015.10.009

**Description**

**CROSS-REFERENCE TO RELATED APPLICATIONS**

This application claims the benefit under 35 U.S.C. § 119(e) of U.S. Provisional Application No. 62/893,703, filed August 29, 2019, titled "BIOMARKER-AGNOSTIC COLORIMETRIC SIGNATURE ARRAY DISTINGUISHES BETWEEN MULTIPLE ANALYTES AND DISEASE STATES".

**TECHNICAL** FIELD

The present disclosure relates generally to systems, devices, and methods of using detectable arrays. More specifically, the present disclosure relates to optimized systems, devices, and methods of detecting diverse types of analytes, and diagnosing disease states based on the detection of such analytes, using detectable arrays that comprise photoinitiators or photocleavage products thereof.

**BACKGROUND**

Detectable arrays can be used to bind or immobilize an analyte on a surface or feature for detecting presence of analytes and diagnosing disease states. Many existing detectable arrays are limited to selectively bind to a single analyte or single type of analyte, which limits their application to samples containing multiple analytes of interest. Methods of analyzing binding of analytes to detectable arrays for diagnosing disease states are also limited, as they typically require that analytes be either labeled before they are captured on a surface (such as an array), for example by addition of dye, stain, ligand, etc., or they must be bound by a labeled-probe (e.g., oligonucleotide, antibody, labeled antibody, etc.) after binding to the surface. These methods suffer from many limitations including label bias and added expense due to labeling reactions, background caused by non-specific labeling. Thus, there is a need in the art for improved methods for the unbiased capture and detection of unlabeled analytes.
Patent documents CA2923228 A1 and US2017/175169 A1 represent background art.

**SUMMARY OF INVENTION**

The present disclosure provides, *inter alia,* optimized systems, devices, and methods of capturing on a substrate including a plurality of features (e.g., an array), and detecting with or without a label, a plurality of different types of analytes.
A method is provided in accordance with the invention that includes: receiving, at a processor and for a plurality of subjects, image data associated with colorimetric or luminescence signal profiles of a plurality of array spots (1) contacted with one or more samples associated with that subject and (2) heated at a predetermined temperature for a predetermined period of time, each of the plurality of array spots including a different hydrogel composition with a photoinitiator that was previously exposed to ultraviolet (UV) light; processing, for each of the plurality of subjects, the image data associated with that subject to produce color intensity values for each of the plurality of array spots by summing red, green, and blue pixel intensities for each of the plurality of array spots; training, for each of the plurality of subjects, a neural network to classify that subject by calibrating the neural network using the color intensity values associated with each subject other than that subject and of the plurality of subjects, the neural network including a multilayer perceptron including an input layer having about 210 nodes, the number representing 70 spots multiplied by three color channels, and a hidden layer having between about 30 nodes to 250 nodes; and predicting, for each of the plurality of subjects, a diagnostic class for that subject using the neutral network trained for that subject.
In an embodiment, a method of determining disease, disorder, or condition state in a subject includes: contacting an array with one or more analytes from the subject; incubating the one or more analytes on the array for a first period of time, thereby allowing a portion of the one or more analytes to be captured on the array; heating the array at a predetermined temperature for a second period of time; and measuring, using an imaging device, an amount of one or more colorimetric or luminescence signals produced in response to the heating.

**BRIEF DESCRIPTION OF THE DRAWINGS**

The patent or application file contains at least one drawing executed in color. Copies of this patent or patent application publication with color drawing(s) will be provided by the Office upon request and payment of the necessary fee.

FIG. 1 is a schematic illustration of a detectable array, according to some embodiments.
FIG. 2 is a schematic illustration of a process of one or more analytes binding to a detectable array, according to some embodiments.

FIG. 3 depicts a reaction scheme of photocleavage of 2,2-Dimethoxy-2-phenylacetophenone (DMPA) into molecular species benzyl and benzoin.

FIG. 4A depicts different solutions with and without DMPA and having been exposed or not exposed to ultraviolet (UV) light.

FIG. 4B is a line graph depicting absorbance of different solutions of DMPA with various volumetric ratios of UV light exposed and non-UV light exposed DMPA.

FIGS. 5A, 5B, and 5C are bar graphs depicting relative signals of colorimetric products produced by different monomers containing DMPA while varying different parameters. FIG. 5A depicts changes in the relative signals based on different UV exposure times. FIG. 5B depicts changes in the relative signals based on different DMPA concentrations. FIG. 5C depicts changes in the relative signals based on temperature.

FIGS. 6A-6C show bar graphs depicting relative signals of colorimetric products produced by different monomers containing different photoinitiators and at different temperatures.

FIG. 7 depicts *p*-values of *t*-tests performed on data collected from photoinitiator experiments depicted in FIGS. 5A-5C and 6.

FIG. 8 is a flow chart depicting a method of applying a sample to an array and obtaining colorimetric data from the array, according to some embodiments.

FIG. 9 is a flow chart depicting a method of preparing a detectable array with a photoinitiator, according to some embodiments.

FIG. 10 is a flow chart depicting a method of analyzing colorimetric data obtained from an array, according to some embodiments.

FIGS. 11A and 11B depict colorimetric profiles of arrays exposed to thirty-nine (39) different analytes.

FIG. 12 depicts a spot intensity heat map of the colorimetric products of the arrays exposed to the thirty-nine (39) different analytes depicted in FIGS. 11A and 11B.

FIG. 13 depicts the output of performing hierarchical clustering on the colorimetric data of the arrays exposed to the thirty-nine (39) different analytes depicted in FIGS. 11A and 11B.

FIG. 14 depicts graphs showing different computational analyses of the colorimetric data of the arrays exposed to the thirty-nine (39) different analytes depicted in FIGS. 11A and 11B.

FIGS. 15A-15D are graphs showing the demographic profile of subjects in a disease state study using arrays, according to some embodiments.

FIG. 16 depicts a flow showing inputs and outputs from a model trained using colorimetric data produced by arrays, according to some embodiments.

FIG. 17A depicts performance metrics for the model, as depicted in FIG. 16.

FIG. 17B depicts information regarding misclassification of the model, as depicted in FIG. 16.

FIG. 17C are charts depicting true positives and sensitivity of different stages of cancer across different sample sizes, using analyses and models, as applied to the subjects in the disease state study depicted in FIGS. 15A-15D, according to some embodiments.

FIG. 17D is a line graph depicting sensitivity of different stages of cancer across different sample sizes, using analyses and models, as applied to the subjects in the disease state study depicted in FIGS. 15A-15D, according to some embodiments.

FIG. 18 is a flow chart depicting a method of training a model and classifying or diagnosing samples using the model, as applied in the disease state study depicted in FIGS. 15A-15D, according to some embodiments.

FIG. 19 depicts colorimetric profiles of arrays exposed to twenty-nine (29) different analytes and an array exposed to no analyte.

FIG. 20 depicts an example compute device for processing and analyzing colorimetric data, according to some embodiments.

## DETAILED DESCRIPTION

Systems, devices, and methods described herein relate *inter alia* to capturing and detecting different analytes using detectable arrays and detection methods. In some embodiments, the present disclosure provides detectable arrays comprising photoinitiators or photocleavage products thereof. In some embodiments, analytes (e.g. labeled or unlabeled analytes) are detected on the detectable arrays using non-enzymatic browning reaction. In some embodiments, the present disclosure provides methods of diagnosing diseases or conditions in a subject utilizing the detectable arrays to detect one or more analytes present in a sample obtained from the subject. In some embodiments, subjects diagnosed as having a disease or condition, according to such methods of diagnosing, are treated with a suitable treatment.

In embodiments described herein, systems, devices, and methods can employ, unless indicated specifically to the contrary, conventional methods of molecular biology, recombinant DNA techniques, protein expression, and protein / peptide / carbohydrate chemistry within the skill of the art, many of which are described below for the purpose of illustration.

Examples of such techniques are described in Sambrook, et al., Molecular Cloning: A Laboratory Manual (3rd Edition, 2000); DNA Cloning: A Practical Approach, vol. I & II (D. Glover, ed.); Oligonucleotide Synthesis (N. Gait, ed., 1984); Oligonucleotide Synthesis: Methods and Applications (P. Herdewijn, ed., 2004); Nucleic Acid Hybridization (B. Hames & S. Higgins, eds., 1985); Nucleic Acid Hybridization: Modern Applications (Buzdin and Lukyanov, eds., 2009); Transcription and Translation (B. Hames & S. Higgins, eds., 1984); Animal Cell Culture (R. Freshney, ed., 1986); Freshney, R.I. (2005) Culture of Animal Cells, a Manual of Basic Technique, 5th Ed. Hoboken NJ, John Wiley & Sons; B. Perbal, A Practical Guide to Molecular Cloning (3rd Edition 2010); Farrell, R., RNA Methodologies: A Laboratory Guide for Isolation and Characterization (3rd Edition 2005). Poly(ethylene glycol), Chemistry and Biological Applications, ACS, Washington, 1997; Veronese, F., and J.M. Harris, Eds., Peptide and protein PEGylation, Advanced Drug Delivery Reviews, 54(4) 453-609 (2002); Zalipsky, S., et al., "Use of functionalized Poly(Ethylene Glycols) for modification of polypeptides" in Polyethylene Glycol Chemistry: Biotechnical and Biomedical Applications.

**Overview**

The present disclosure can provide *inter alia* arrays for capturing diverse analytes, e.g., from complex biological mixtures, and methods for label-free detection of such analytes as well as a diagnostic platform based on the same.

The present disclosure is based in part on the surprising discovery that, in some embodiments, the inclusion of optimized concentrations of photoinitiator compounds (e.g., 2,2-dimethoxy 2-phenylacetophenone (DMPA), 1-hydroxy-cyclohexyl phenyl ketone (HCPK), and 2-hydroxy-2-methyl-1-phenyl-1-propanone (HMPP), and photocleavage products thereof) in hydrogel arrays prior to UV-based polymerization results in macromolecule polymers that are optimal for capturing analytes and detecting the same via non-enzymatic browning reactions (e.g., via caramelization or Maillard reactions). The present disclosure is also based in part on the surprising discovery that, in some embodiments, certain optimal lengths of exposure to UV light during hydrogel array polymerization results in hydrogel arrays that enable improved colorimetric detection of bound analytes via non-enzymatic browning reactions (e.g., via caramelization or Maillard reactions). The present disclosure is also based in part on the surprising discovery that, in some embodiments, heating such hydrogel arrays comprising photoinitiator compounds, or photocleavage products thereof, at certain optimal temperatures for certain optimal times after contacting the arrays with one or more analyte produces improved colorimetric signatures that are sufficiently differentiated and enable classification of samples including different types or amounts of analytes. Thus, the present disclosure provides *inter alia* improved and versatile detectable arrays and methods of using the same to capture and detect analytes without the need for analyte labeling.

In an embodiment, a plurality of monomer, polymer, or monomer and polymer stock solutions are prepared, mixed with a photoinitiator compound, and placed in contact with a substrate. In an embodiment, the plurality of monomer, polymer, or monomer and polymer stock solutions are prepared, mixed with a photoinitiator compound, and spotted onto a substrate to form arrays. The monomer, polymer, or monomer and polymer stock solutions may be mixed together by means of the spotting on the arrays, or they may be mixed together prior to spotting on the arrays. In some aspects, the substrate comprises a substrate coating. In some aspects, the substrate coating comprises one or more appropriate chemical groups for fixing one or more macromolecules to the surface of the substrate. For example, in particular embodiments, the substrate coating comprises chemical groups that can be polymerized along with monomers or polymers spotted on the substrate. In some aspects, the arrays including a plurality of monomer, polymer, or monomer and polymer stock solution mixed with a photoinitiator, are exposed to UV light for a period of time to enable curing of any monomers and polymers spotted thereon with the photoinitiator and the substrate coating, e.g., to facilitate covalent attachment of the monomers and polymers to the substrate and/or to facilitate photocleavage of the photoinitiator into free radicals that react with other free radicals to form new molecular species which are trapped within macromolecules. After polymerization, the arrays can be exposed to different samples comprising one or more analytes (e.g., from samples obtained from a subject) thereby capturing one or more analytes from the samples on the array. The arrays comprising the captured analytes may subsequently be heated at a certain temperature to produce colorimetric signatures, which can be captured using an imaging device. In some aspects, colorimetric signatures produced by the arrays and captured using an imaging device are processed and analyzed using a computational model (e.g., neural network) to classify the samples (e.g., to diagnose a disease or condition).

In some embodiments, systems, devices, and methods described herein are used to diagnose different types of diseases or conditions and/or different stages of disease (e.g., different stages of cancer) or a condition. Conventional approaches to diagnosis typically require identification and validation of new biomarkers for detecting and classifying disease. Such identification and validation of biomarkers is a rate limiting step for medical diagnostics. Complex signatures integrating multiple analytes can be one approach to address this limitation. Current signature platforms, however, tend to be focused on specific analyte classes such as, for example, nucleic acids, antibodies, proteins or volatile organic compounds. Such sample-based specificity requires sample processing steps to isolate and label the target analyte class. Specialized equipment (e.g., DNA sequencers, microarray scanners, and mass spectrometers), therefore, are often required to isolate the analytes and/or enable detection of the results. Therefore, such signature platforms can be limited in the analytes they can analyze and require significant sample processing and specialized knowledge of equipment that makes such platforms difficult to use.

Systems, devices, and methods described herein provide signature platforms that address a chemical space that covers multiple analyte classes, such as, for example, nucleic acids, antibodies, proteins or volatile organic compounds. In some embodiments, use of such signature platforms comprise sample contact with hydrogel arrays, as described herein, and heating of such arrays. Such signature platforms can therefore reduce costs, be easy to use, and enable simultaneous screening of multiple conditions.

In some embodiments, systems, devices, and methods can utilize urine samples, which is an under-utilized source of diagnostic information. Despite the advantages of urine, the market for hematologic testing is greater than urinalysis. Urine contains defined analytes that can arrive in a collection vessel pre-filtered and ready for analysis without requiring further processing. Collection of urine is non-invasive and less prone to sample degradation, unlike blood samples, which can suffer from hemolysis. In addition, analyses based on the detection of ctDNA and other circulating nucleic acids can be highly specific and lacking in sensitivity. Such liquid biopsy approaches can require additional layers of information, such as epigenetics and proteomics, to increase sensitivity. Accordingly, systems, devices, and methods described herein that make use of urine profiles with hydrogel arrays can be a useful complement to liquid biopsy approaches.

In some embodiments, systems, devices, and methods described herein can be customized for certain contexts. For example, analyte signatures can be used as a general screen for diseases, to localize a disease site (e.g., a cancer site), to detect recurrence of disease, or as a screening test for a single disease. In particular, urine signatures can be used to screen for diseases such as, for example, lung cancer, for which no generally available screening test currently exists. In the U.S., for example, a computerized tomography (CT) scan can be recommended for individuals at high risk of lung cancer because of the harm that results from radiation and the high false positive rate or such scans.

In some embodiments, systems, devices, and methods described herein can be used to detect different stages of a disease, e.g., different stages of cancer. The present disclosure is based in part on the surprising discovery that cancer at different stages can have distinct colorimetric signatures that can be distinguished using computational methods described herein. In some embodiments, the distinct signatures can be used to distinguish indolent from lethal cancers. Such data can be useful in minimizing the over-diagnosis and over-treatment of slow growing tumors that have no effect on a patient's life expectancy.

Systems, devices, and methods can reduce the need for human interpretation and therefore human training and/or knowledge. In some embodiments, signature platforms as described herein can be based on a data-driven approach. Such signature platforms can capture complex dependencies and correlations within colorimetric data using computational systems and methods. Such complex dependencies and correlations can be lost when a single or few biomarkers are focused on. In some embodiments, such platforms can be used to identify biomarkers to illuminate the molecular mechanisms underlying a disease.

It should be noted that embodiments and features described in the context of one of the aspects or embodiments of the present invention also apply to the other aspects and embodiments of the invention.

**Arrays**

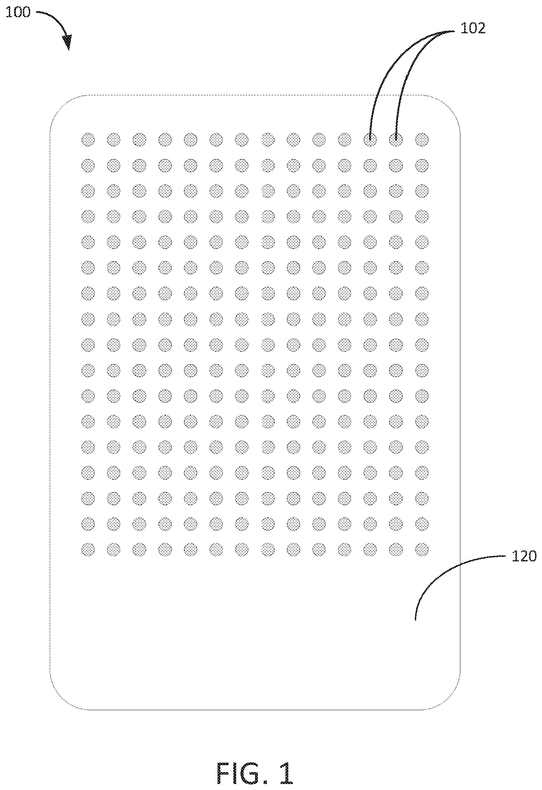

A detectable array may comprise a substrate with a plurality of features or binding sites for binding one or more analytes. FIG. 1 depicts a non-limiting example of a detectable array 100, according to some embodiments. The detectable array 100 includes a substrate 120 with a plurality of features 102. As can be appreciated by a person of skill in the art, the number of features that may be included on the array can be limited by the size of the substrate and the desired density of the features, but that different sizes of substrate and/or density can produce a smaller or greater number of features 102. The substrate 120 can be made of any suitable substrate material, such as, e.g., one or more of plastic, glass, and ceramic. In certain embodiments, the substrate is glass. In particular embodiments, the substrate is silicate or borosilicate glass. The plurality of features 102 can have any appropriate shape or size, depending on the shape or size of the substrate. For example, when the substrate 120 is a plate, the plurality of features 102 can be wells in the plate. When the substrate 120 is a slide, each of the plurality of features 102 can be a different location on the slide. In alternative embodiments, when the substrate is a particle, the plurality of features can be pore or openings in the particle. In one embodiment, the substrate 120 comprises at least one of, or one or more of, glass, plastic, metal, composites, acrylics, or biologically active substrates.

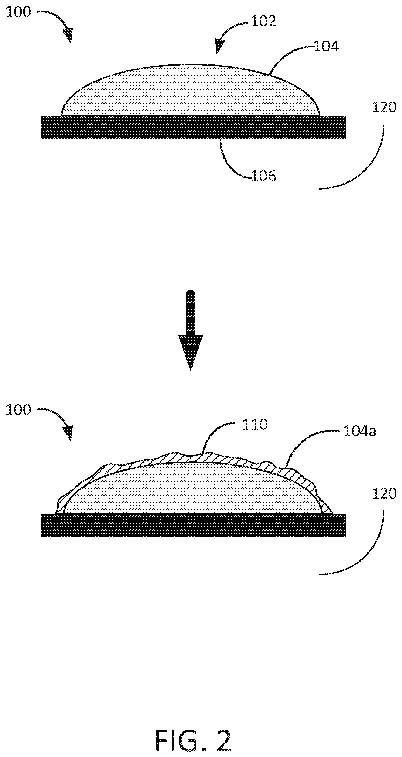

FIG. 2 depicts a cross-sectional view of an example feature 102 of the detectable array 100, according to embodiments. Each of the plurality of features 102 can independently comprise one or more substrate coatings 106 for fixing one or more macromolecules 104 to the surface of the substrate 120. The one or more substrate coatings 106 can, independently, coat all or a portion of each of the plurality of features. The substrate coatings 106 can be any coatings that contain appropriate chemical groups for fixing one or more macromolecules 104 to the surface of the substrate. In some embodiments, the identity of the substrate coatings 106 can depend on the identity of the one or more macromolecules 104 to be affixed to the substrate. For example, if the one or more macromolecules 104 include nanotubes, then the substrate coating 106 can have a chemical moiety that binds to nanotubes. As another example, if the one or more macromolecules 104 includes a polymer, then the substrate coating 106 can have one or more functional groups that can polymerize into the polymer backbone, such as olefin. In some embodiments, the substrate coating 106 includes at least one silane or at least one siloxane. Particular siloxanes utilized in some embodiments include one or more acrylosiloxanes, such as one or more of 3-methacryloxypropyl trimethoxy silane, 3-acryloxypropyl trimethoxy silane, N-(3-acryloxy-2-hydroxypropyl)-3-aminopropyltriethoxysilane, and 3-methacryloxy propyldimethylchlorosilane.

The one or more macromolecules (e.g., 104 on exemplary detectable array 100) can include any macromolecules. In some particular embodiments, the macromolecules provide one or more unbiased binding sites. Such macromolecules with unbiased binding sites can, in some embodiments, have one or more free functional groups for binding analytes, such as carbonyls, amines, amides, carboxylic acids, esters, alcohols, and the like. For example, some polymer-based macromolecules used in the present invention have one or more free functional groups for binding analytes, such as carbonyls, amines, amides, carboxylic acids, esters, alcohols, and the like. Additionally, polymers with or without free functional groups can bind one or more analytes based not only on the nature of the functional groups (if present), but also based on the shape of the polymers and their interaction with the analytes in three dimensions, e.g., by virtue of their size and shape. Similarly, macromolecules comprising nanotubes may, or may not contain any free functional groups, but can still bind one or more analytes by virtue of their size and shape.

The one or more macromolecules (e.g., 104 on exemplary detectable array 100) can be, for example, at least one of polymer, surfactant, nanosphere, nanotube, dendrimer, microsphere, and polymerized microsphere. When the macromolecules include polymer, the polymer can be a homopolymer or copolymer. The macromolecules 104 can comprise one or more of (meth)acrylamides, (meth)acrylates, and N,N'-(alkylene)bisacrylamide. For example, in some embodiments, the macromolecules 104 comprise one or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-dimethyl-3-oxobutyl)acrylamide, N-isopropylacrylamide, ethylene glycol phenyl ether acrylate, N,N'-methylenebisacrylamide, 1, 1,3,3,3-hexafluoroisopropyl acrylate, and N-tert-octylacrylamide. In some embodiments, the macromolecules comprise two or more of 2-carboxyethyl acrylate, acrylic acid, acrylamide, histamine acrylate, N-[tris(hydroxymethyl)methyl]acrylamide, hydroxypropyl acrylates, 4-hydroybutyl acrylate, N-hydroxyethyl acrylamide, N,N,-dimethylacrylamide, N-(1,1-dimethyl-3-oxobutyl)acrylamide, N-isopropylacrylamide, ethylene glycol phenyl ether acrylate, N,N'-methylenebisacrylamide, 1, 1,3,3,3-hexafluoroisopropyl acrylate, and N-tert-octylacrylamide.

In some embodiments, the one or more macromolecules (e.g., 104 on exemplary detectable array 100) can also comprise at least one cross-linker. The cross-linker can be any cross-linker known in the art. Cross-linkers can include, for example, one or more molecules containing two, three, four, or more olefins or acrylic functional groups, such as one or more of bis-acrylamide, trimethylolpropane triacrylate, bisphenol A-bis(2-hydroxypropyl)acrylate, and 1-(acryloyloxy)-3-(methacryloyloxy)-2-propanol.

The one or more macromolecules (e.g., 104 on exemplary detectable array 100) can be arranged on the substrate in a predetermined shape or pattern. For example, as shown on detectable array 100, a plurality of macromolecules may be deposed evenly along the x and y axis of the detectable array creating an ordered plurality of macromolecules each separated from the nearest neighboring macromolecule by the same distance. In some embodiments, it is desirable to optimize the spacing between the macromolecules, e.g., to ensure that during detection, signals from any analytes captured by one macromolecule to not interfere with the signals from analytes captured by a nearby macromolecule. In some embodiments, the spacing of the macromolecules on the detectable array is not even, but varies along one or more portions of the detectable array. In some embodiments, each of the plurality features (e.g., the plurality of features 102 on exemplary detectable array 100) comprises a different macromolecule. In some embodiments, several of the plurality of features (e.g., the plurality of features 102 on exemplary detectable array 100) comprise similarly or identical macromolecules. In some embodiments, such redundancy of macromolecules presented on the detectable array can be beneficial, as it enables replicate capture and detection of analytes on a single array, which can increase the reliability and reproducibility of analyte detection using such arrays. Thus, each substrate coating 106 of each of the plurality of features can contain macromolecules that are unique in either chemical identity, three-dimensional shape, pattern of physical disposition, or a combination thereof, with respect to the other features of the substrate. Or, the detectable array may comprise a plurality of each of such unique macromolecules, thereby providing redundancy on the array to enable replicate detection of analytes on a single array. These variables can be accomplished in a number of ways. For example, lithographic techniques can be used to create different patterns of macromolecules on the different features. As another example, when the substrate is a plate, the macromolecules fixed to each well of the plate can have different chemical identities. As a further example, when the substrate 120 is a slide as depicted in FIG. 1, each chemically distinct macromolecule can be affixed to a different location on the slide; in this case each of the different locations on the slide is a different feature, such that the slide comprises a plurality of features 102 with chemically distinct macromolecules affixed thereto. In these or other manners, the one or more macromolecules 104 can comprise a plurality of chemically distinct macromolecules, and each chemically distinct macromolecule can be affixed to a different feature 102 of the plurality of features 102 of the substrate 120. For example, the one or more macromolecules 104 can comprise at least two, at least twelve, at least seventy-two, at least ninety-six, or at least two-hundred and eighty-eight chemically distinct macromolecules, each of which can be affixed to a different feature of the plurality of features of the substrate.

In some embodiments, as further described below, the macromolecules 104 can include hydrogels cured with photoinitiators. The hydrogels can be formed by combining one or more monomer stock solutions and a photoinitiator solution, and then curing the combined solutions with ultraviolet (UV) light. For example, a macromolecule 104 can be formed by combining a single monomer with a photoinitiator. Alternatively, different macromolecules 104 can be formed by polymerizing two monomers, e.g., A and B, together at different ratios (e.g., 50:50, 10:90). In some instances, having different ratios of monomers can lead to differences in structure and differential binding of analytes.

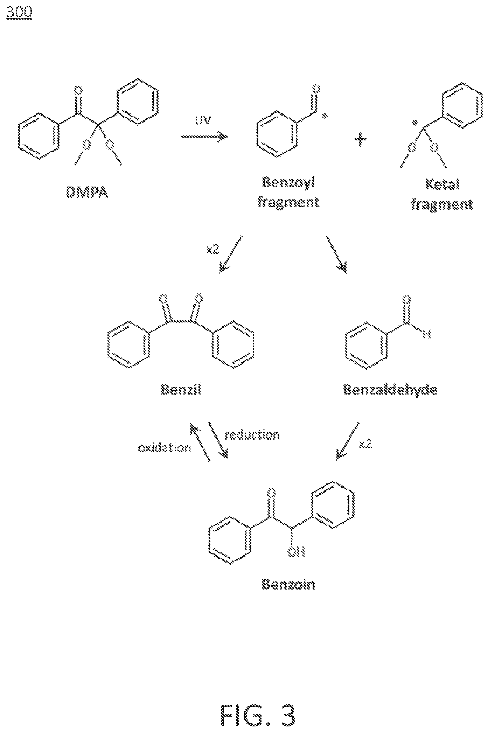

Examples of suitable monomers include: acrylamide, 2-carboxyethyl acrylate, acrylic acid, 2-cyanoethyl acrylate, N-[tris(hydroxymethyl)methyl] acrylamide, hydroxypropyl acrylate isomers, 4-hydroxybutyl acrylate, N-hydroxyethyl acrylamide, N,N-dimethylacrylamide, N-isopropylacrylamide, N-(1,1-dimethyl-3-oxobutyl) acrylamide, 2-methacryloxyethyl phenyl urethane, 1-acryloyloxy-3-(methacryloyloxy)-2-propanol and ethylene glycol phenyl ether acrylate. The monomer stock solutions can be prepared using methods known in the art. For example, in some embodiments, monomer stock solutions are prepared by dissolving a monomer (e.g., one of the suitable monomers listed above) in a suitable solvent. For example, in some embodiments, dimethyl sulfoxide (DMSO) is used to dissolve one or more of such monomers. In some embodiments, monomer stock solutions are prepared by dissolving a monomer in a monomer solvent solution disclosed herein (e.g., Monomer Solvent 1 or Monomer Solvent 2) either directly or after first dissolving the monomer in dimethyl sulfoxide (DMSO). For example, in some embodiments, a monomer is dissolved in DMSO and then further mixed with a monomer solvent solution containing acrylamide, N,N'-methylenebisacrylamide, distilled water, and DMSO, and then additional acrylic acid or acrylamide is added as required to achieve desired concentrations of monomer. The photoinitiator solution can be formulated by dissolving the photoinitiator in any suitable solvent. In particular embodiments, the photoinitiator is dissolved in DMSO. Examples of suitable photoinitiators DMPA, HCPK, and HMPP. The hydrogel solutions can be placed (e.g., spotted) onto a substrate (e.g., substrate 120) and subsequently cured using UV light. Further details regarding the specific parameters of certain hydrogel preparations and UV curing parameters are described with reference to FIGS. 3-6.

In some embodiments, when a detectable array such as the detectable array 100 is placed in contact with a sample containing analytes, one or more macromolecules 104 on the detectable array 100 bind to one or more analytes 110. In some embodiments, the analytes bind to a surface 104a of the one or more macromolecules 104. In some embodiments, the analytes are able to diffuse inside the macromolecules via pores created during the polymerization process, and the analytes then bind inside the macromolecules. The one or more analytes 110 can comprise one or more of small molecules, proteins, peptides, nucleotides, nucleosides, bacteria, viruses, fungi cells, yeast cells, and animal cells bound to at least one of the one or more macromolecules. In particular, a plurality of different analytes 110 can be bound to the one or more macromolecules. For example, if the detectable array 100 is contacted with the blood or urine of a subject, the one or more macromolecules 104 can bind to cells, such as red blood cells, white blood cells, t-cells, and the like, and also bind to proteins and small molecules that are present in the blood. In particular, when each of the plurality of features 102 has a different affixed macromolecules, different types and quantities of analytes 110 can bind to each of the plurality of features, thereby creating a detectable pattern of bound analytes.

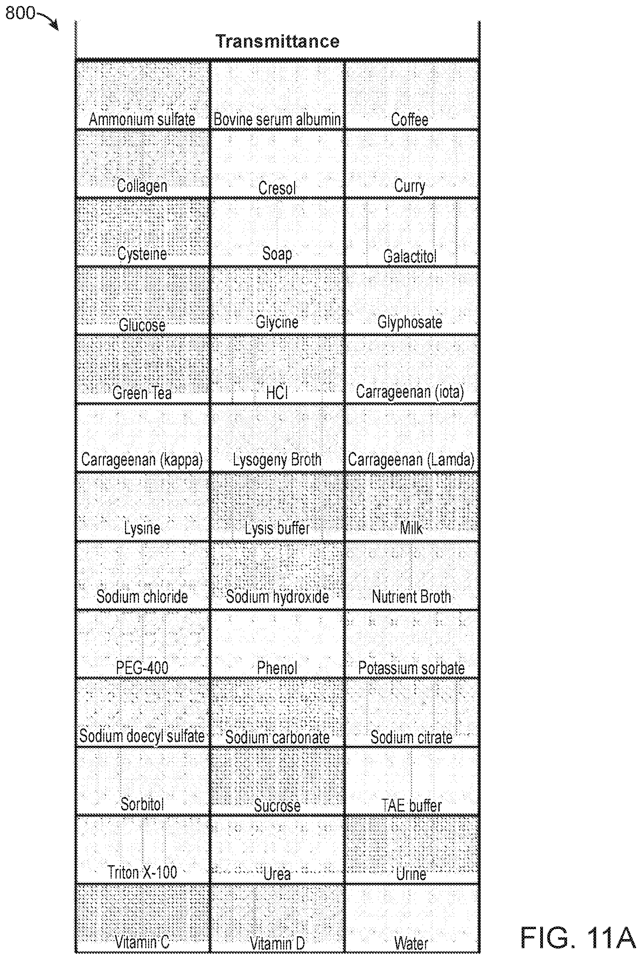

The presence or absence of one or more analytes 110 bound to the one or more macromolecules 104 can be detectable by a plurality of detection methods, as further described herein. Exemplary detection methods include one or more of Maillard reaction, caramelizing, reaction with one or more amine reactive dyes, reaction with one or more thiol reactive dyes, reaction with one or more cellular dyes, reaction with one or more solvatochromic dyes, reaction with one or more acid indicators, reaction with one or more base indicators, reaction with one or more labeled antibodies, luminescence, surface texture analysis, photo-scanning, microscopy, photo-scanning with reflectance or transmittance illumination, photography with reflectance or transmittance illumination, mass spectrometry and spectroscopy. Further details with respect to detecting using one or more detection methods, e.g., Maillard reactions and caramelizing, are described below with reference to the examples below and FIGS. 11A-18.

## Photoinitiators

Systems, devices, and methods described herein can provide an analyte-agnostic approach using arrays including hydrogels cured with a photoinitiator. Suitable examples of photoinitiators for hydrogel arrays as described herein include DMPA, HCPK, and HMPP.

Spray reagents provide a way to visualize organic compounds on Thin Layer Chromatography (TLC) plates. Spray reagents can include photocleavage products of photopolymerization initiators or photoinitiators. By incorporating the chemical reactivity of photoinitiators into a combinatorially-diverse array of hydrogels, such arrays can provide colorimetric signatures that can enable the classification of samples without prior knowledge of specific anlaytes bound to the array. Such an approach can involve heating the array to produce a visual colorimetric output, but can avoid the need for more costly modes of data acquisition such as, for example, mass spectrometry.

In an embodiment, DMPA can be used for photo-polymerizing acrylamide and acrylate-based materials. Photocleavage of DMPA results in the production of a benzoyl and a ketal fragment, which can further dimerize or cross-react to create multiple photolysis products. Two such products are benzil and benzoin (i.e., a reduced form of benzil), both of which can be used as TLC spray visualization reagents. FIG. 3 depicts a general reaction scheme of DMPA showing the production of benzyl and benzoin.

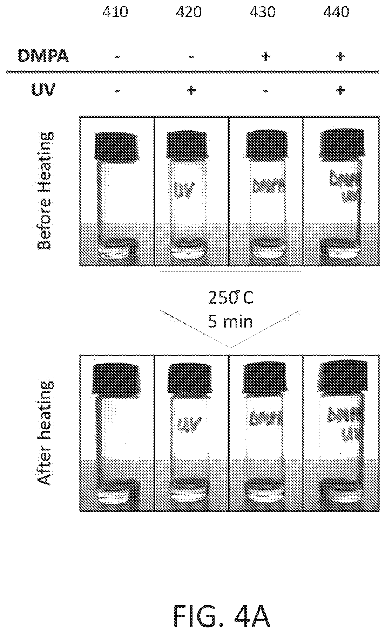

Experiments were conducted to evaluate the effect of UV cleavage on DMPA and the colorimetric signals produced by resulting molecules. FIG. 4A depicts the results of a first experiment. In the first experiment, a DMPA solution was prepared by dissolving 30.6 mg of DMPA in 400uL of DMSO and adding this solution to 3.6mL of mineral oil in a 15mL tube. The mixture was vortexed vigorously for a few seconds. 400uL of the DMPA-mineral oil mixture was then added to each of two glass vials. One vial 440 was exposed to UV light for 60 minutes using a Biorad UV transilluminator with a wavelength of 302 nm. The other vial 430 was used as a non-UV exposed control. Additionally, controls without DMPA were also prepared by adding 400uL of DMSO to 3.6mL of mineral oil and vortexing vigorously for a few seconds. In a similar fashion, 400uL of the DMSO-mineral oil mixture was then added to each of two glass vials. One vial 420 was exposed to UV light for 60 minutes and the other 410 was used as a non-UV control. All 4 samples were then heated on a hot plate at 250°C for 5 minutes, and subsequently cooled to room temperature for 30 minutes. FIG. 4A shows the DMPA solution pre-exposed to UV (i.e., vial 440) producing yellow products upon heating, while none of the other samples produced significant visually observable colorimetric signals.

In a second experiment, a DMPA solution was prepared by dissolving 1.5g of DMPA in 6ml of DMSO. Two glass vials of 700uL DMPA were prepared and one was exposed to UV light (390 nm) for one hour while the other was not exposed to UV light. Various ratios of the non-UV-exposed DMPA solution (UV-) and UV-exposed DMPA solution (UV+) were prepared according to Table 1:

**Table 1**

| UV-:UV+ ratio | 1:0 | 2:1 | 1:1 | 1:2 | 0:1 | 0:0 |
|---|---|---|---|---|---|---|
| UV- DMPA (uL) | 100 | 67 | 50 | 33 | 0 | 0 |
| UV+ DMPA (uL) | 0 | 33 | 50 | 67 | 100 | 0 |
| DMSO (uL) | 100 | 100 | 100 | 100 | 100 | 200 |
| Mineral Oil (mL) | 3.8 | 3.8 | 3.8 | 3.8 | 3.8 | 3.8 |
| Final vol (mL) | 4.0 | 4.0 | 4.0 | 4.0 | 4.0 | 4.0 |

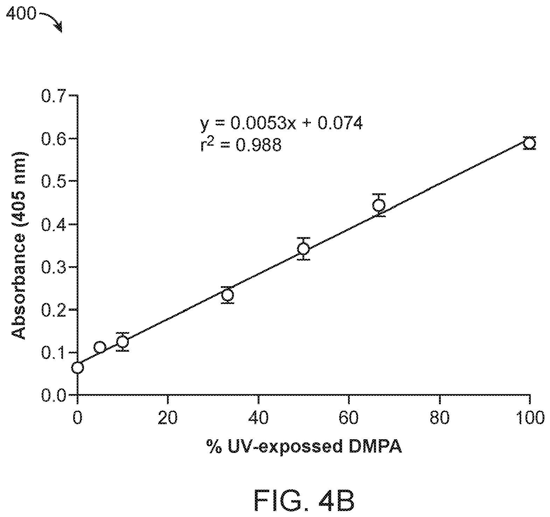

FIG. 4B is a graph 400 depicting the amount of heated, colorimetric products (as measured by absorbance) of mixtures containing the various ratios of UV- DMPA solution and UV+ DMPA solution shown in Table 1. Graph 400 shows that UV cleavage of DMPA can produce molecules with colorimetric properties upon heating. As shown in FIG. 4B, the colorimetric products produced by the mixtures increases in linear proportion to the relative percentage of UV-exposed DMPA. With the experimental setup associated with the second experiment, this linear relationship can be represented by $y = 0.0053x + 0.074$, where y represents the absorbance of the mixture at 405 nm and x represents the percentage of UV-exposed (i.e., UV+) solution in the mixture.

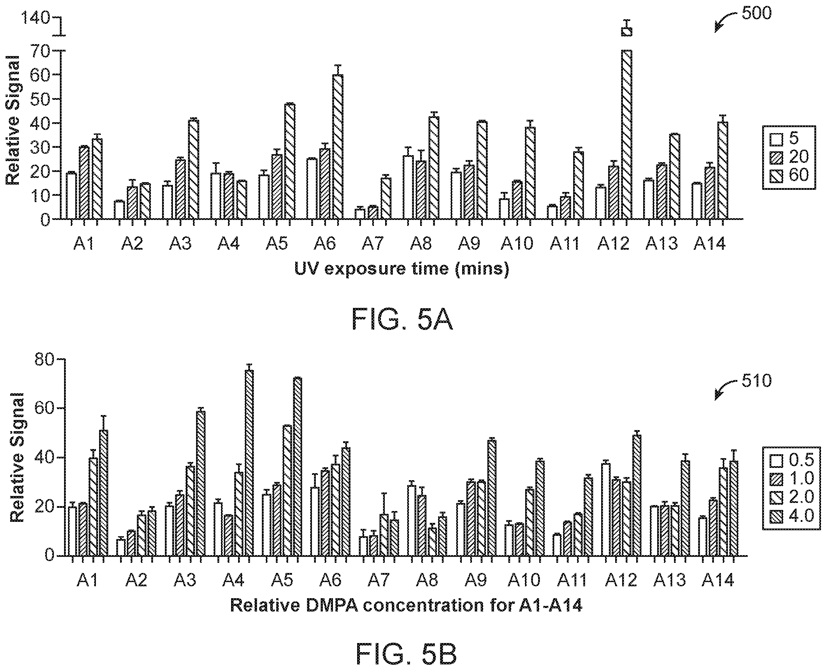

Multiple factors can affect the properties of the colorimetric products produced using hydrogels cured with DMPA. These factors can include, for example, UV exposure time, DMPA concentration, and heating temperature. These factors were tested using a prototype spot array of fourteen different hydrogels mounted on a microscope slide and photocured with DMPA under different conditions. FIGS. 5A-5C depict the results of the testing, with the fourteen different hydrogels labeled A1 to A14 and the relative signal of the color intensity of the hydrogels obtained by using a flatbed scanner in a transmittance mode to capture the spot intensities of the hydrogels and suitable software to analyze the spot intensities.

The fourteen hydrogels A1 to A14 were prepared using the following working solutions, monomer stock solutions, and photoinitiator solutions.

Working solutions:

**Monomer Solvent 1 (MS-1)**

| | |
|---|---|
| Acrylamide | 38 mg |

(continued)

| N,N'-methylenebisacrylamide | 29 mg |
|---|---|
| Distilled Water | 0.8 mL |
| DMSO | 1.134 mL |

**Monomer Solvent 2 (MS-2)**

| Acrylamide | 38 mg |
|---|---|
| N,N'-methylenebisacrylamide | 29 mg |
| DMSO | 1.934 mL |

**2M Acrylamide**

| Acrylamide | 0.142 g |
|---|---|
| DMSO | 108 uL |
| MS-1 | 750 uL |

The hydrogels A1 to A14 comprised between one and three monomers selected from a group of fourteen monomers including: acrylamide, 2-carboxyethyl acrylate, acrylic acid, 2-cyanoethyl acrylate, N-[tris(hydroxymethyl) methyl] acrylamide, hydroxypropyl acrylate isomers, 4-hydroxybutyl acrylate, N-hydroxyethyl acrylamide, N,N-dimethylacrylamide, N-isopropylacrylamide, N-(1,1-dimethyl-3-oxobutyl) acrylamide, 2-methacryloxyethyl phenyl urethane, 1-acryloyloxy-3-(methacryloyloxy)-2-propanol and ethylene glycol phenyl ether acrylate.

Monomer stock solutions M1 and M15 were formulated by dissolving the monomer first in DMSO and either MS-1 or MS-2, and then adding additional acrylic acid or acrylamide as required to the final concentrations stated in Table 2 below. MS-1 was used as the solvent for M1 to M14, and MS-2 was used for M15.

**Table 2**

| No. | Monomer | Monomer Quantity | DMSO (uL) | Monomer Solvent (uL) | Additional Acrylic Acid final conc. (M) | Additional Acrylamide final conc. (M) | Monomer final conc. (M) |
|---|---|---|---|---|---|---|---|
| M1 | Acrylamide | 142 mg | 108 | 750 | | | 2 |
| M2 | 2-Carboxyethyl acrylate | 242 uL | 8 | 750 | | | 2 |
| M3 | Acrylic Acid | 144 uL | 106 | 750 | | | 2 |
| M4 | Acrylic Acid + 0.5% NaOH | 144 uL | 81 | 750 | | | 2 |
| M5 | 2-Cyanoethyl acrylate | 182 uL | | 618 | | 0.4 | 1.6 |
| M6 | N-[tris(hydroxymethyl) meth y] acrylamide | 175 mg | | | | 1 | 1 |
| M7 | Hydroxypropyl acrylate, isomers | 250 uL | | 750 | | | 2 |
| M8 | 4-hydroxybutyl acrylate | 288.4 uL | | 712 | | | 2 |
| M9 | N-Hydroxyethyl acrylamide | 208 uL | 42 | 750 | | | 2 |
| M1 1 | N-iso-propylacrylamide | 226 mg | 24 | 750 | | | 2 |

(continued)

| No. | Monomer | Monomer Quantity | DMSO (uL) | Monomer Solvent (uL) | Additional Acrylic Acid final conc. (M) | Additional Acrylamide final conc. (M) | Monomer final conc. (M) |
|---|---|---|---|---|---|---|---|
| M1 2 | N-(1,1-Dimethyl-3-oxobutyl) acrylamide | 255 mg | | 500 | | 0.5 | 1.5 |
| M1 3 | 2-methacryloxyethyl phenyl urethane | 42.2 mg | 57.8 | | 1.8 | | 0.2 |
| M1 4 | 1-Acryloylox-y)-3(methacryloy-loxy)-2-propanol | 28 uL | 25 | 365 | 1 | 1 | 0.15 |
| M1 5 | Ethylene glycol phenyl ether acrylate | 420 uL | | 580 | | | 2 |

The photoinitiator solutions were made by first dissolving the photoinitiator in DMSO to make a stock solution and then constituting the relevant photoinitiator solutions as described below.

**PI solution (for DMPA or HCPK)**

| | |
|---|---|
| DMPA or HCPK (0.255 g/mL in DMSO) | 100 uL |
| Glycerol | 80 uL |
| Distilled Water | 400 uL |
| DMSO | 3.42 mL |

**PI solution (for HMPP)**

| | |
|---|---|
| HMPP (1.04 g/mL in DMSO) | 30 uL |
| Glycerol | 80 uL |

| | |
|---|---|
| Distilled Water | 400 uL |
| DMSO | 3.49 mL |

Table 3 provides the proportion by volume of each monomer stock solution for each polymer used in the hydrogels A1 to A14.

**Table 3**

| | | Polymers A1 to A14 | | | | | | | | | | | | | |
|---|---|---|---|---|---|---|---|---|---|---|---|---|---|---|---|
| | | A1 | A2 | A3 | A4 | A5 | A6 | A7 | A8 | A9 | A1 0 | A1 1 | A1 2 | A1 3 | A1 4 |
| **Monomer Stock Solution (uL)** | M1 | 10 | | | | | | | | | | | | | |
| | M2 | | 10 | | | | | | | | | | | | |
| | M3 | | | 10 | | | | | | | | | | | |
| | M4 | | | | 10 | | | | | | | | | | |
| | M5 | | | | | 10 | | | | | | | | | |

(continued)

| | | Polymers A1 to A14 | | | | | | | | | | | | |
|---|---|---|---|---|---|---|---|---|---|---|---|---|---|---|
| | | A1 | A2 | A3 | A4 | A5 | A6 | A7 | A8 | A9 | A10 | A11 | A12 | A13 | A14 |
| | M6 | | | | | | 10 | | | | | | | | |
| | M7 | | | | | | | 10 | | | | | | | |
| | M8 | | | | | | | | 10 | | | | | | |
| | M9 | | | | | | | | | 10 | | | | | |
| | M10 | | | | | | | | | | 10 | | | | |
| | M11 | | | | | | | | | | | 10 | | | |
| | M12 | | | | | | | | | | | | 10 | | |
| | M13 | | | | | | | | | | | | | 10 | |
| | M14 | | | | | | | | | | | | | | 10 |

For the arrays, borosilicate glass slides were initially washed three times with distilled water, followed by sonication in absolute ethanol for five minutes to remove surface contaminants. Slides were blow dried then dried in an oven at 120°C for five minutes followed by coating with 3-methacryloxypropyltrimethoxylsilane through vapor deposition overnight at 93°C and 580 mmHg. Thereafter, slides were dried in an oven at 120°C for one hour.

For each polymer solution A1 to A14, 10uL of DMPA PI solution was added to 10uL of each solution A1 to A14 and mixed with a pipet. 1.5uL of each solution was spotted onto a glass slide, as appropriately silanized according to the above procedures, and polymerized with a 302 nm UV transilluminator (e.g., Biorad 2000 UV transilluminator). UV light exposure catalyzes both the curing of the polymer and also the covalent attachment of the polymers onto the silanized glass surface.

FIG. 5A depicts a graph 500 of the relative signals of the fourteen hydrogels A1 to A14 at different UV exposure times. In the experiment, the hydrogel arrays were UV cured for incrementing durations, i.e., 5, 20, and 60 minutes, before heating them at 250°C for 5 minutes. In graph 500, the first bar (i.e., left most bar) above each hydrogel label A1 through A14 corresponds to the relative signal of the hydrogel cured for 5 minutes, the second bar (i.e., middle bar) above each hydrogel label A1 through A14 corresponds to the relative signal of the hydrogel cured for 20 minutes, and the third bar (i.e., right most bar) above each hydrogel label A1 through A14 corresponds to the relative signal of the hydrogel cured for 60 minutes.

The arrays were digitally scanned for image analyses. Specifically, slides were scanned with a digital scanner (e.g., EPSON Perfection v500) at 1200 dpi and stored as images. Each image was converted to 8-bit grayscale and inverted. For each spot associated with a hydrogel, a central circular region of standardized area was selected suing an area selection tool and analyzed. Mean intensity values were measured for the central circular region of each spot. Background mean intensity values were also measured for each array. The spot mean intensity values were then adjusted based on the background mean intensity values and plotted against the factors of interest, i.e., UV exposure time, DMPA concentration, heating temperature, and/or photoinitiator species (i.e., DMPA, HCPK, HMPP). Each data point was generated and analyzed in triplicate.

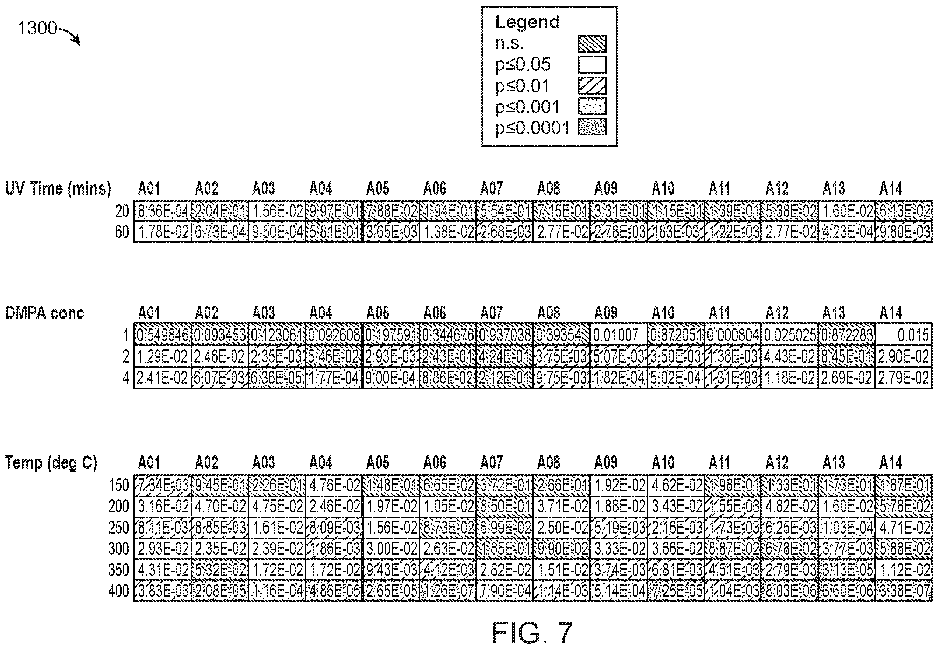

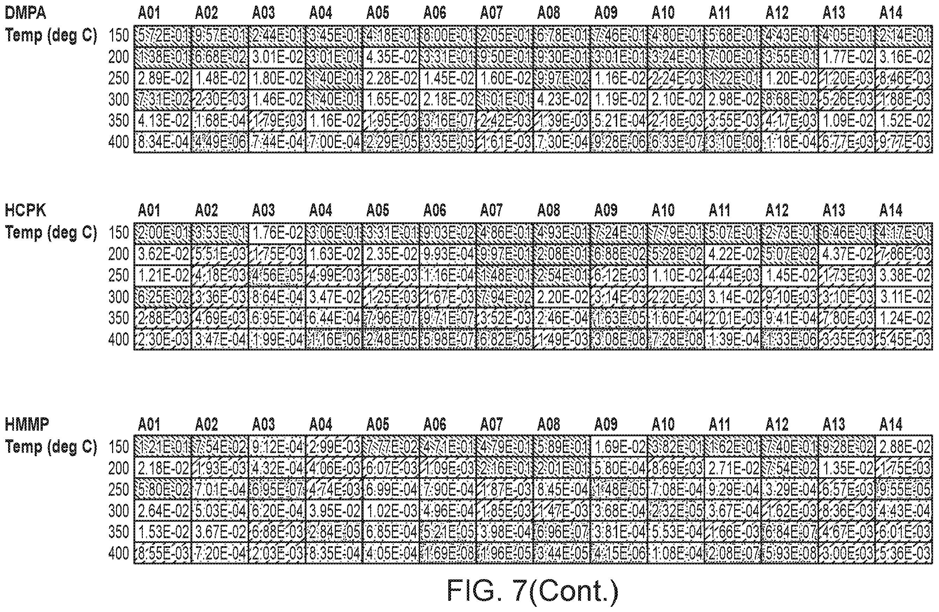

As depicted in graph 500, each of the hydrogel spots with the exception of A4 demonstrates an increase in color intensity with increasing UV exposure time. The signal change between 5 and 60 minutes of UV curing was statistically significant for each of the hydrogel spots with the exception of A4, as depicted in FIG. 7, further described below.

FIG. 5B depicts a graph 510 of the relative signals of the fourteen hydrogels A1 to A14 at different DMPA concentrations. In the experiment, hydrogel arrays were polymerized with 0.5x, 1x, 2x, or 4x DMPA, as detailed below, with UV exposure of 60 minutes and subsequently heated at 250°C for 5 minutes.

| | 0.5X DMPA | 1X DMPA | 2X DMPA | 4X DMPA |
|---|---|---|---|---|
| DMPA Solution | 50uL | 100uL | 200uL | 400uL |
| Glycerol | 80uL | 80uL | 80uL | 80uL |
| Distilled Water | 400uL | 400uL | 400uL | 400uL |
| DMSO | 3.47mL | 3.42mL | 3.32mL | 3.07mL |

The arrays were digitally scanned for image analyses, as described above. In graph 510, the first bar (i.e., left most bar) above each hydrogel label A1 through A14 corresponds to the relative signal of the hydrogel with 0.5x DMPA, the second bar (i.e., left middle bar) above each hydrogel label A1 through A14 corresponds to the relative signal of the hydrogel with 1x DMPA, the third bar (i.e., right middle bar) above each hydrogel label A1 through A14 corresponds to the relative signal of the hydrogel with 2x DMPA, and the fourth bar (i.e., right most bar) above each hydrogel label A1 through A14 corresponds to the relative signal of the hydrogel with 4x DMPA. Graph 510 depicts each of the hydrogel spots with the exception of A8 demonstrating an increase in color intensity with increasing relative DMPA concentrations. The signal change with increasing DMPA concentrations from 0.5x to 4x DMPA was statistically significant for each of the hydrogel spots with the exception of A6 and A7, as depicted in FIG. 7, further described below.

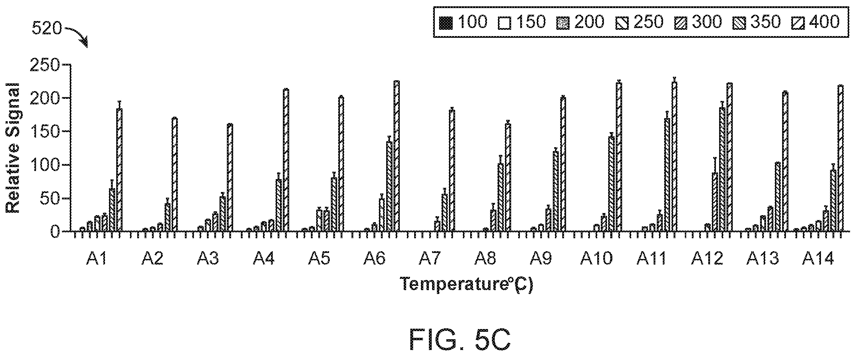

FIG. 5C depicts a graph 520 of the relative signals of the fourteen hydrogels A1 to A14 heated at different temperatures. In the experiment, hydrogel arrays polymerized with UV light for 60 minutes were subsequently heated using a heated plate for 5 minutes at different temperatures ranging from 100°C to 400°C in 50°C increments (i.e., heated at 100°C, 150°C, 200°C, 250°C, 300°C, 350°C or 400°C). The surface temperature of the heated plate was verified by an infrared thermometer during the heating process. The arrays were digitally scanned for image analyses, as described above. Graph 520 depicts increasing relative signals for each of the hydrogels with increasing temperature. In graph 520, the bars above each hydrogel label A1 through A14 correspond to the relative signal of the hydrogel with starting at 100°C on the left and increasing to 400°C on the right. Compared to the signal increases of graphs 500 and 510 (i.e., UV exposure time and DMPA concentration, respectively), the signal increases associated with heating temperature produced the greatest and most significant changes. Accordingly, the experiments demonstrate that heating temperature is the limiting factor for the creation of colorimetric signals in hydrogels cured with UV light. The experiments further demonstrate the principle that colorimetric signals can be created in hydrogels using a photoinitiator. The first two experiments (i.e., UV exposure time and DMPA concentration) further indicate that photocleavage products contribute to the production of such colorimetric signals in the hydrogels.

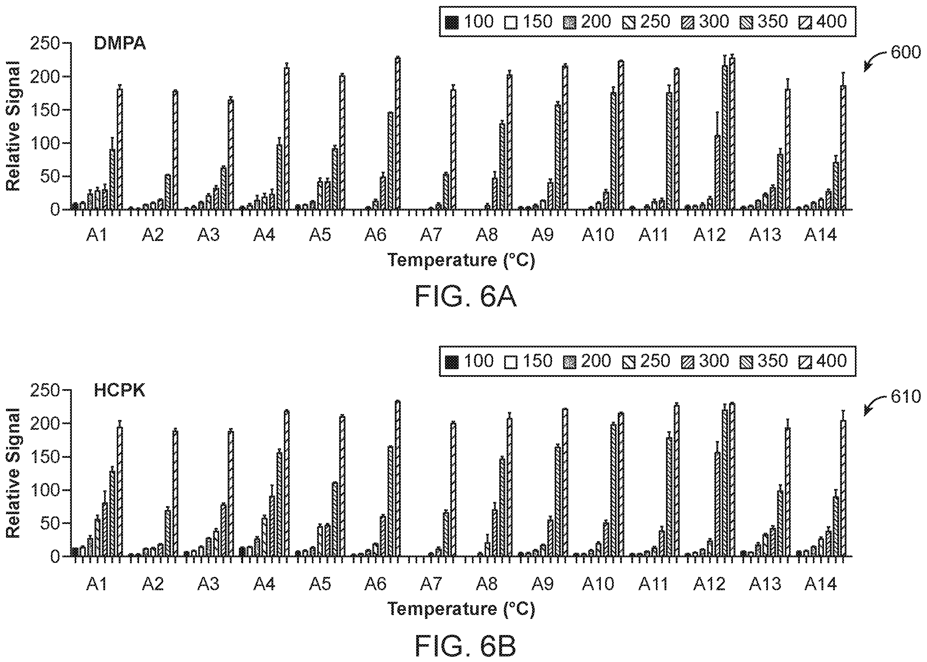

To further test the effect of heating temperature on the relative colorimetric signal production of hydrogels cured with a photoinitiator, an experiment was conducted that heated hydrogel arrays including different photoinitiators at temperatures ranging from 100°C to 400°C (i.e., at 100°C, 150°C, 200°C, 250°C, 300°C, 350°C or 400°C). The arrays were digitally scanned for image analyses, as described above. FIGS. 6A-6C depict the results of this experiment conducted with three different photoinitiators, DMPA, HCPK, and HMPP. Graph 600 corresponds to the results obtained using DMPA, graph 610 corresponds to the results obtained using HCPK, and graph 620 corresponds to the results obtained using HMPP. Graphs 600, 610, and 620 show that each of the three photoinitiators demonstrated a similar pattern of temperature-dependent signal increase. Spot intensities of the hydrogels were significantly greater at higher temperatures, e.g., 350°C, compared to lower temperatures, e.g., 100°C, regardless of the photoinitiator.

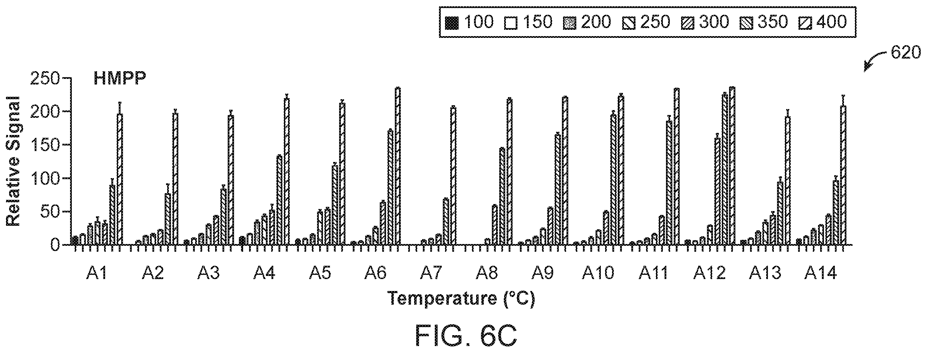

FIG. 7 depicts the statistical analysis of the data in FIGS. 5A-6. The statistical significance of the data was evaluated using a $t$-test (2-tailed, heteroscedastic) to compare each data point from the respective results shown in FIGS. 5A-6 against its relevant starting condition. The resulting $p$-values from the $t$-tests are shown in chart 1300 in FIG. 7, with the statistical significance of the data being evaluated based on the $p$-values. For example, $p$-values less than or equal to 0.05 indicated that the difference between a particular data point and its starting condition is statistically significant. For UV exposure time (FIG. 5A), each data point associated with higher UV exposure times was compared against the data point associated with UV exposure at 5 minutes. Accordingly, graph 1300 depicts the $p$-values from the $t$-tests conducted with data points associated with 20 minutes and 60 minutes of UV exposure compared to the data point associated with 5 minutes of UV exposure. For DMPA concentration (FIG. 5B), each data point associated with higher concentrations of DMA was compared against the data point associated with 0.5x DMPA. Accordingly, graph 1300 depicts the $p$-values from the t-tests conducted with data points associated with 1x, 2x, and 4x DMPA compared to the data point associated with 0.5x DMPA. For heating temperature (FIGS. 5C and 6), each data point associated with the higher temperatures was compared against the data point associated with 100°C. Accordingly, graph 1300 depicts the $p$-values from the $t$-tests conducted with data points associated with 150°C, 200°C, 250°C, 300°C, 350°C, and 400°C compared to the data point associated with 100°C.

In some embodiments, an array includes a plurality of spotted solutions, each including one or more macromolecules mixed with a photoinitiator. In some embodiments, the one or more macromolecules can include one or more monomers selected from the group consisting of: acrylamide, 2-carboxyethyl acrylate, acrylic acid, 2-cyanoethyl acrylate, N-[tris(hydroxymethyl)methyl] acrylamide, hydroxypropyl acrylate isomers, 4-hydroxybutyl acrylate, N-hydroxyethyl acrylamide, N,N-dimethylacrylamide, N-isopropylacrylamide, N-(1,1-dimethyl-3-oxobutyl) acrylamide, 2-methacryloxy-yethyl phenyl urethane, 1-acryloyloxy-3-(methacryloyloxy)-2-propanol and ethylene glycol phenyl ether acrylate. In some embodiments, the one or more macromolecules can include one or more monomers selected from the group consisting of: 2-carboxyethyl acrylate, acrylic acid, acrylamide, histamine acrylate, N-[tris(hydroxymethyl)methyl]acrylamide, hydroxypropyl acrylate isomers, 4-hydroybutyl acrylate, N-hydroxyethyl acrylamide, N,N,-dimethylacrylamide, N-(1,1-dimethyl-3-oxobutyl)acrylamide, N-isopropylacrylamide, ethylene glycol phenyl ether acrylate, N,N'-methylenebisacrylamide, 1,1,3,3,3-Hexafluoroisopropyl acrylate, and N-tert-octylacrylamide. In some embodiments, the plurality of spotted solutions can include a homopolymer including one monomer, a heteropolymer including at least two monomers, a heteropolymer including at least three monomers, or any number or combinations thereof.

In some embodiments, the substrate includes a surface that is functionalized. In some embodiments, the substrate includes a surface that is functionalized with a silane or siloxane coating. In some embodiments, the substrate includes a surface that is functionalized with acrylosiloxane, e.g., selected from methacryloxypropyl trimethoxy silane, 3-acryloxypropyl trimethoxy silane, N-(3-acryloxy-2-hydroxypropyl-3-aminopropyltriethoxysilane, 3-methacryloxy propyldimethylchlorosilane, and any combination thereof.

Examples of suitable photoinitiators include DMPA, HCPK, and HMPP. In some embodiments, the concentration of photoinitiator can be consistent across the plurality of solutions. In alternative embodiments, individual or subsets of solutions can have the photoinitiator concentrations that differ from other individual or subsets of solutions. In some embodiments, each of the plurality of solutions can have a concentration of photoinitiator between about 0.5x (6.25nM) and about 4x (50 nM), including all ranges and values therebetween. In some embodiments, each of the plurality of solutions can have a concentration of photoinitiator of about 1x (12.5nM).

In some embodiments, the array can include about fourteen spots, about seventy spots, including all ranges and values therebetween. In some embodiments, each spot can include about 1uL, about 1.3uL, about 1.5uL, about 2uL, including all ranges and values therebetween.

In some embodiments, the array including the plurality of spotted solutions can be polymerized using UV light for a predetermined period of time. In some embodiments, the UV light can have a wavelength of between about 250 nm to about 400 nm. In some embodiments, the UV light can have a wavelength of between about 300 nm to about 350 nm. In some embodiments, the UV light can have a wavelength of about 250 nm, about 302 nm, about 350 nm, about 390 nm, about 400 nm, inclusive of all ranges therebetween. In some embodiments, the predetermined period of time for UV light exposure can be about 5 minutes, about 20 minutes, about one hour, about two hours, about several hours, inclusive of all ranges and values therebetween.

In some embodiments, the polymerized array can be exposed to a sample including one or more analytes (labeled or unlabeled) and incubated for a predetermined period of time. In some embodiments, the arrays can be incubated with the sample for about 5 minutes, about 10 minutes, about 15 minutes, including all ranges and values therebetween. After incubating with the sample, the polymerized array can be heated at a predetermined temperature for a predetermined period of time. In some embodiments, the array can be heated at about 100°C, about 150°C, about 200°C, about 250°C, about 300°C, about 350°C, about 400°C, including all ranges and values therebetween. In some embodiments, the array can be heated at a single temperature, while in other embodiments, the array can be heated at multiple temperatures for equal or different periods of time. In some embodiments, the array can be heated at the predetermined temperature for about 1 minute, about 5 minutes, about 10 minutes, about 15 minutes, including all ranges and values therebetween. In some embodiments, the array can be heated to a temperature from about 200°C to about 400°C, such as about 275°C to about 325°C or about 300°C, for sufficient time to induce a Maillard reaction, such as from about 1 min to about 15 min, or about 5 min. In some embodiments, the array can be heated to a temperature from about 100°C to about 210°C, such as about 130°C to about 210°C, or about 160°C to about 200°C, for a sufficient time to cause caramelization. In some embodiments, the arrays can be heated via a hot plate.

In an embodiment, a hydrogel array suitable for disease detection applications as described herein can include a plurality of hydrogel spots each polymerized with about 1x DMPA (12.5 mM) with about 20 minutes of UV light exposure and capable of being heated at a temperature of as high as about 250°C.

## Methods

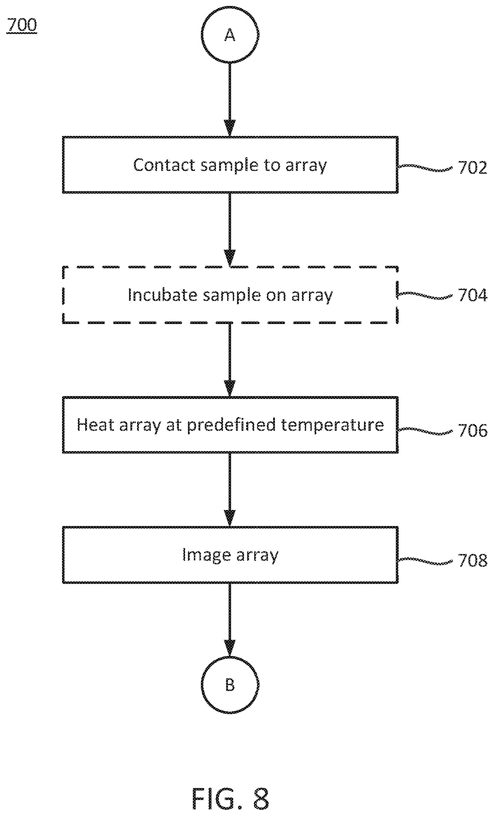

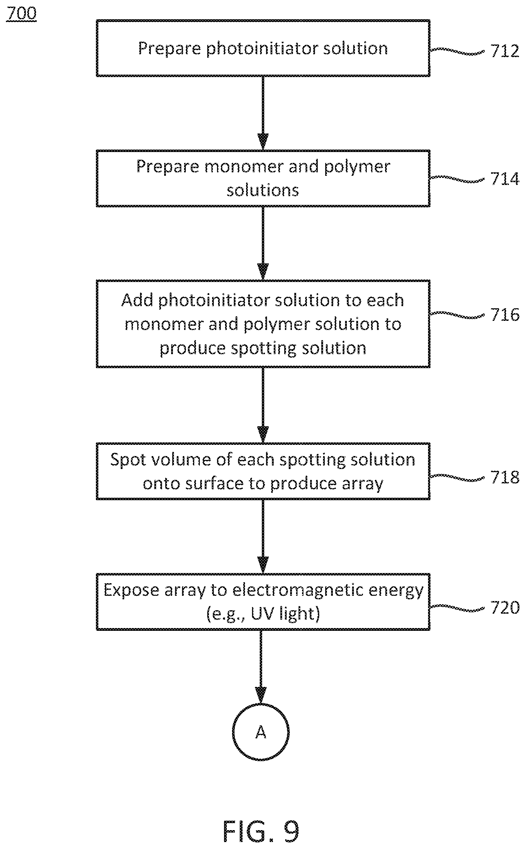

FIGS. 8-10 are flow diagrams of a method 700 of detecting an analyte (e.g., a labeled or unlabeled analyte) and classifying a sample. As depicted in FIG. 8, the method 700 can include contacting one or more samples with an array (e.g., array 100), at 702. The one or more samples can include unlabeled or labeled analytes, or a combination of labeled and unlabeled analytes. In some embodiments, the samples can be urine samples. The array can be a hydrogel array, e.g., an array including one or more monomers or polymers and one or more photoinitiators (e.g., DMPA, HCPK, HMPP), that was previously exposed to UV light for a first predetermined period of time, as further depicted and described with reference to FIG. 9. Optionally, the array can be incubated with the sample for a second predetermined period of time (e.g., between about 5 minutes to about 15 minutes), at 704. The array with the sample can be heated at a predefined temperature (e.g., between about 100°C to about 400°C), at 706, and be imaged (e.g., using a flatbed scanner or other imaging device), at 708. As described herein, when the array is heated and/or illuminated, the array can produce colorimetric data (e.g., a colorimetric signal) that can be captured by an imaging device.

FIG. 9 depicts preparation of the array, prior to contact with a sample, at 702. In some embodiments, preparation of the array can include steps that are similar to preparation of the photoinitiator solution mixed with the monomer stock solutions M1 to M15, as described herein. For example, one or more photoinitiator solutions can be prepared, at 712. Preparation of the photoinitiator solution can include, for example, dissolving a photoinitiator (e.g., DMPA, HCPK, HMPP) in a solution containing DMSO, distilled water, and glycerol. In an embodiment, amounts of photoinitiator, glycerol, distilled water, and DMSO can be:

**PI solution (e,g., for DMPA or HCPK)**

| | |
|---|---|
| DMPA or HCPK (0.255 g/mL in DMSO) | 100 uL |
| Glycerol | 80 uL |
| Distilled Water | 400 uL |
| DMSO | 3.42 mL |

**PI solution (e.g., for HMPP)**

| | |
|---|---|
| HMPP (1.04 g/mL in DMSO) | 30 uL |
| Glycerol | 80 uL |
| Distilled Water | 400 uL |
| DMSO | 3.49 mL |

One or more monomer and polymer solutions can be prepared, at 714. In some embodiments, preparation of such solutions can include preparing a plurality of monomer stock solutions, e.g., similar to the preparation of monomer stock solutions M1 through M15, as described herein. For example, each monomer stock solution can be prepared by dissolving a monomer in a solution including one or more of DMSO, acrylamide, N,N'-methylenebisacrylamide, or distilled water, and optionally adding additional acrylic acid or acrylamide as necessary to achieve desired concentrations of the monomer. Examples of suitable monomer stock solutions (e.g., monomer stock solutions M1 to M15) are depicted in Table 2 above. One or more polymer solutions can then be prepared using the monomer stock solutions, e.g., by combining different amounts of monomer stock solutions, as depicted, for example, in Tables 3-12. The photoinitiator solution can be added to each monomer and polymer solutions to produce spotting solutions, at 716. In some embodiments, the monomer and polymer solutions can be mixed with the photoinitiator solutions using a pipet. In some embodiments, the final concentrations of the photoinitiator can be between about 0.5x (6.25 mM) and about 4x (50 mM).

Each spotting solution can be spotted onto a surface of a substrate, at 718, and exposed to electromagnetic energy in the form of UV light, at 720. In some embodiments, the substrate can be, for example, borosilicate glass. In some embodiments, the borosilicate glass can be prepared by washing each slide with distilled water, followed by sonication in absolute ethanol to remove surface contaminants. The slides can then be air dried and/or dried with heat (e.g., in an oven), coated with a substrate coating (e.g., 3-methacryloxypropyltrimethoxylsilane), and dried again. The UV light can be at a wavelength of about 250nm to about 400nm. In some embodiments, the array can be exposed to UV light for the first predetermined period of time, e.g., between about 5 minutes and about 60 minutes. Exposure of the spotting solution can result in several outcomes for the photoinitiator. In some instances, the photoinitiator is photocleaved by the UV into free radicals and these free radicals participate in polymerizing the monomers or polymers and can become incorporated into terminal ends of the monomers or polymers in the process. In some instances, the photoinitiator is photocleaved by UV into free radicals that do not participate in polymerization but react with other free radicals to form new molecular species, which in turn are able to react with analytes to produce a colorimetric signal. And in some instances, residual photoinitiator can escape photocleavage. Systems, devices, and methods described herein predominately rely on the photocleaved products of the photoinitiators to produce the colometric signals described herein for detection and/or diagnostic purposes. After the UV exporesure, the method 700 can then proceed to 702, i.e., where a sample (e.g., including analytes) is contacted with the array.

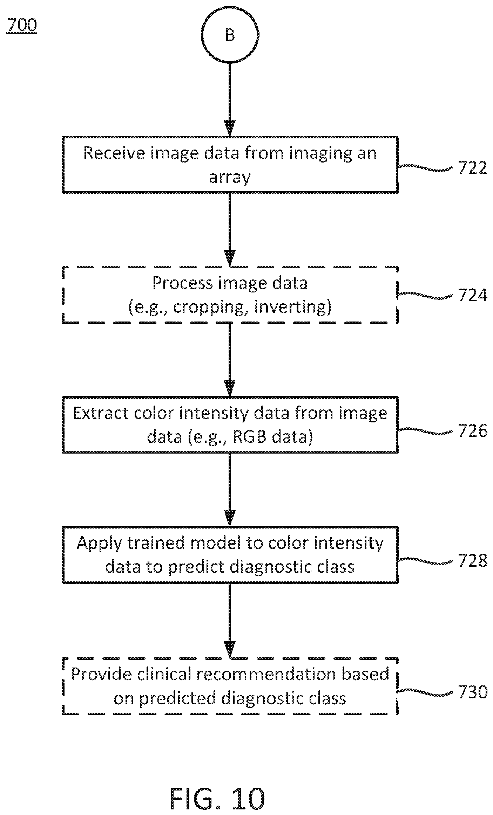

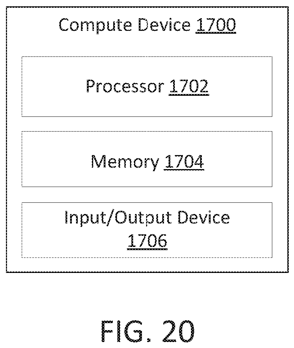

FIG. 10 depicts processing and analysis of image data (e.g., JPEG) captured by an imaging device (e.g., flatbed scanner, analog or digital imaging device, etc.) of the array, at 708. Such processing can be conducted, e.g., by a compute device or one or more processors of such as compute device 1700, as depicted in FIG. 20. As depicted in FIG. 20, compute device 1700 can include a processor 1722, a memory 1724, and an input/output device 1726. While one processor 1722, memory 1724, and input/output device 1726 are depicted, it can be appreciated that any number of processors, memory, and input/output devices can be included in compute device 1700 such that compute device 1700 is configured to provide the functionality described herein. The compute device 1700 can be operatively coupled to other components of a signature platform, as described herein. For example, compute device 1700 can be coupled to one or more of an imaging device, a heating device (e.g., a heating plate), etc. to acquire image data and/or control operation of such components and/or devices.

The processor 1702 can be any suitable processing device configured to run and/or execute a set of instructions or code and may include one or more data processors, image processors, graphics processing units, physics processing units, digital signal processors, and/or central processing units. In embodiments described herein, the processor 1702 can be any suitable processing device configured to run and/or execute functions associated with processing and/or analyzing image data, including applying one or more trained models, to classify a sample. The processor 1702 can be, for example, a general purpose processor, Field Programmable Gate Array (FPGA), an Application Specific Integrated Circuit (ASIC), and/or the like. The processor 1702 can be configured to run and/or execute application processes and/or other modules, processes and/or functions associated with the system and/or a network associated therewith. The underlying device technologies may be provided in a variety of component types (e.g., metal-oxide semiconductor field-effect transistor (MOSFET) technologies like complementary metal-oxide semiconductor (CMOS), bipolar technologies like emitter-coupled logic (ECL), polymer technologies (e.g., silicon-conjugated polymer and metal-conjugated polymer-metal structures), mixed analog and digital, and the like.

The memory 1704 can include a database (not shown) and may be, for example, a random access memory (RAM), a memory buffer, a hard drive, an erasable programmable read-only memory (EPROM), an electrically erasable read-only memory (EEPROM), a read-only memory (ROM), Flash memory, and/or the like. The memory 1704 can store instructions to cause the processor 1702 to execute modules, processes, and/or functions associated with processing and/or analyzing image data. Some embodiments described herein relate to a computer storage product with a non-transitory computer-readable medium (also may be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also may be referred to as code or algorithm) may be those designed and constructed for the specific purpose or purposes.

Examples of non-transitory computer-readable media include, but are not limited to, magnetic storage media such as hard disks, floppy disks, and magnetic tape; optical storage media such as Compact Disc/Digital Video Discs (CD/DVDs); Compact Disc-Read Only Memories (CD-ROMs), and holographic devices; magneto-optical storage media such as optical disks; solid state storage devices such as a solid state drive (SSD) and a solid state hybrid drive (SSHD); carrier wave signal processing modules; and hardware devices that are specially configured to store and execute program code, such as Application-Specific Integrated Circuits (ASICs), Programmable Logic Devices (PLDs), Read-Only Memory (ROM), and Random-Access Memory (RAM) devices. Other embodiments described herein relate to a computer program product, which may include, for example, the instructions and/or computer code disclosed herein.

The input/output device 1706 can include an input device (e.g., keyboard, touch screen, audio device) and/or an output device (e.g., display device, audio device). The input/output device 1706 can include a communication interface that enables compute device 1700 to interface with one or more components of a signature platform or system, as described herein. For example, the communication interface can be configured to receive data from an imaging device and/or send signals (e.g., generated by processor 1702) to one or more of an imaging device, a heating device, a light source (e.g., a UV light or illuminator), etc. to control operation of those devices. In some embodiments, the processor 1702 via the communication interface of input/output device 1706 can send signals to activate a UV light (e.g., to apply UV light to polymerize an array), to activate an imaging device to capture image data or an array, and/or to activate a heating device to heat an array.

In some embodiments, the compute device 1700 can be configured to perform method 700 as depicted in FIG. 10. For example, the image data can be received, at 722. Optionally, the image data can be processed, e.g., cropped, inverted, etc., at 724. For example, the image data can be cropped using a fixed window size about each spot of the array. The cropped image data can be split into various color channels (e.g., color intensity data can be extracted from the image data for each spot), at 726. In some embodiments, the image data can be split into R, G. and B channels, as described herein.

A trained computational model (e.g., a neural network) can be applied to the data associated with the various color channels (e.g., color intensity data or RGB data), at 728. In some embodiments, the model can include fully connected neural networks, which can output a probability of the sample belonging to a certain class from a plurality of classes. An example of a suitable neural network is further described in Example 3 below. In some embodiments, the model can be used to classify the sample into one or more diagnostic classes (e.g., healthy, disease 1, disease 2, etc.). For example, as further described in Example 3 below, the model can be used to classify the sample into diagnostic classes including healthy and one or more types of cancer or disease. Optionally, a clinical recommendation can be provided (e.g., by the compute device based on preset algorithms or rules) based on the class. For example, the compute device can indicate that a patient who provided a sample (e.g., a urine sample) should conduct additional testing to identify and/or treat a disease and/or cancer.

**Examples**

### Example 1 - 39 Analyte Study

Systems, devices, and methods described herein can be used to distinguish between different analytes. In some embodiments, systems, devices, and methods can use transmittance and colorimetric profiles to distinguish between different analytes.

Hydrogel arrays cured with DMPA were tested with the following 39 analytes:

| Analyte | Concentration (w/v or v/v) |
|---|---|
| Distilled Water | NA |
| Sodium Chloride | 1% |
| Sodium Citrate | 1% |
| Sodium Carbonate | 1% |
| Ammonium Sulfate | 1% |
| Potassium Sorbate | 1% |
| HCl | 1% |
| NaOH | 1% |
| Tris Acetate EDTA (TAE) | 1x |
| Urea | 1% |
| Sucrose | 1% |
| Glucose | 1% |
| Galactitol | 1% |
| Sorbitol | 1% |
| PEG400 | 1% |
| SDS | 1% |
| Triton X-100 | 1% |
| Lysis Buffer | 1X |
| Glycine | 1% |
| Lysine | 1% |
| Cysteine | 1% |
| Collagen | 1% |
| Glyphosate (Weed Killer) | 1% |
| Bovine Serum Albumin | 1% |
| Phenol | 1% |
| m-Cresol | 1% |
| Lysogeny Broth (LB) | 1x |
| Nutrient Broth (NB) | 1x |
| Vitamin C | 1% |
| Vitamin D | 1% |
| Green Tea Extract | 0.1% |
| Lambda Carrageenan | 0.1% |
| Kappa Carrageenan | 0.1% |
| Iota Carrageenan | 0.1% |
| Coffee | 0.1% |

(continued)

| Analyte | Concentration (w/v or v/v) |
|---|---|
| Curry Powder | 0.1% |
| Dawn Ultra Soap | 1% |
| Whole Milk | 10% |
| Urine | 40% |

The hydrogel arrays included with 70 spots including varying constituents of hydrogels that provided different combinations of charge, polarity, hydrophobicity, and size exclusion. To prepare the arrays, an equal volume of DMPA photoinitiator solution was added to and mixed with each of the monomer stock solutions A1 to A14 (Table 3), as well as additional stock solutions B1 to B14, C1 to C14, D1 to D14, and E1 to E14, as detailed in Tables 4-7 below.

**Table 4**

| | | Polymers B1 to B14 | | | | | | | | | | | | | |
|---|---|---|---|---|---|---|---|---|---|---|---|---|---|---|---|
| | | B1 | B2 | B3 | B4 | B5 | B6 | B7 | B8 | B9 | B10 | B11 | B12 | B13 | B14 |
| Monomer Stock Solution (uL) | M1 | 7.5 | | | | | | | | | | | | | |
| | M2 | | 7.5 | | | | | | | | | | | | |
| | M3 | | | 7.5 | | | | | | | | | | | |
| | M4 | | | | 7.5 | | | | | | | | | | |
| | M5 | | | | | 7.5 | | | | | | | | | |
| | M6 | | | | | | 7.5 | | | | | | | | |
| | M7 | | | | | | | 7.5 | | | | | | | |
| | M8 | | | | | | | | 7.5 | | | | | | |
| | M9 | | | | | | | | | 7.5 | | | | | |
| | M10 | | | | | | | | | | 7.5 | | | | |
| | M11 | | | | | | | | | | | 7.5 | | | |
| | M12 | | | | | | | | | | | | 7.5 | | |
| | M13 | | | | | | | | | | | | | 7.5 | |
| | M14 | | | | | | | | | | | | | | 7.5 |
| | TFM | 2.5 | 2.5 | 2.5 | 2.5 | 2.5 | 2.5 | 2.5 | 2.5 | 2.5 | 2.5 | 2.5 | 2.5 | 2.5 | 2.5 |

**Table 5**

| | | Polymers C1 to C14 | | | | | | | | | | | | | |
|---|---|---|---|---|---|---|---|---|---|---|---|---|---|---|---|
| | | C1 | C2 | C3 | C4 | C5 | C6 | C7 | C8 | C9 | C10 | C11 | C12 | C13 | C14 |
| | M1 | 4 | | | 4 | | 4 | | | | | | | | |
| Monomer Stock Solution (uL) | M2 | 4 | 4 | | | 4 | | 4 | | 4 | 4 | 4 | | 4 | |
| | M3 | | 4 | | | | | | | 4 | | | | | 2 |
| | M4 | | | 4 | 4 | | | | 4 | | 4 | | | | |
| | M5 | | | 4 | | 4 | 4 | 4 | | | | | 4 | 4 | 2 |
| | M6 | 2 | 2 | | | | | | | | | | 4 | | |
| | M7 | | | 2 | | 2 | | | | | | | | | |
| | M8 | | | | 2 | | | | | | 2 | | | | |

(continued)

| | | Polymers C1 to C14 | | | | | | | | | | | | | |
|---|---|---|---|---|---|---|---|---|---|---|---|---|---|---|---|
| | | C1 | C2 | C3 | C4 | C5 | C6 | C7 | C8 | C9 | C1 0 | C1 1 | C1 2 | C1 3 | C1 4 |
| | M9 | | | | | | 2 | | 4 | | | | | | |
| | M10 | | | | | | | | | | | 4 | | | |
| | M11 | | | | | | | 2 | 2 | 2 | | | | | |
| | M12 | | | | | | | | | | | | | | |
| | M13 | | | | | | | | | | | | | | |
| | M14 | | | | | | | | | | | 2 | 2 | 2 | 6 |

**Table 6**

| | | Polymers D1 to D14 | | | | | | | | | | | | | |
|---|---|---|---|---|---|---|---|---|---|---|---|---|---|---|---|
| | | D1 | D2 | D3 | D4 | D5 | D6 | D7 | D8 | D9 | D1 0 | D1 1 | D1 2 | D1 3 | D1 4 |
| Monomer Stock Solution (uL) | M1 | | | | | | | | | | | | | | |
| | M2 | | 2 | | | | | | | | | | | | |
| | M3 | | | | | | | | 2 | | | | | | |
| | M4 | 2 | | | | | 2 | | | | | | | | |
| | M5 | | | 2 | | 2 | | | | | | 4 | | | |
| | M6 | 4 | | | 4 | 6 | | | | | 4 | | | | 2 |
| | M7 | 4 | 4 | | | | 6 | 4 | | 4 | 2 | 4 | | 4 | |
| | M8 | | 4 | 4 | 4 | | | | 6 | 4 | | | 4 | | |
| | M9 | | | 4 | | | | 4 | | | 4 | | 4 | 4 | 2 |
| | M10 | | | | | 2 | 2 | | | | | | | | |
| | M11 | | | | | | | 2 | 2 | 2 | | | | | |
| | M12 | | | | | | | | | | | | | | |
| | M13 | | | | 2 | | | | | | | | | | |
| | M14 | | | | | | | | | | | 2 | 2 | 2 | 6 |

**Table 7**

| | | Polymers E1 to E14 | | | | | | | | | | | | | |
|---|---|---|---|---|---|---|---|---|---|---|---|---|---|---|---|
| | | E1 | E2 | E3 | E4 | E5 | E6 | E7 | E8 | E9 | E10 | E11 | E12 | E13 | E14 |
| Monomer Stock Solution (uL) | M1 | | | | | | | | | | | | | | |
| | M2 | | | | | | | | | | | | | 2 | |
| | M3 | | | | | | | | | | | | | | |
| | M4 | 2 | 2 | 2 | 2 | 2 | 2 | 2 | 2 | 2 | 2 | | | | |
| | M5 | | | | | | 2 | | | | | | | | |
| | M6 | | | | | | | | | 4 | | | | | |
| | M7 | | | | | | | | | | | | | | |
| | M8 | | | | | | | | 4 | | | | | | |
| | M9 | | | | | | | | | | | | 6 | | |
| | M10 | 4 | | | | 2 | | 6 | | | 4 | | | | |

(continued)

| | | Polymers E1 to E14 | | | | | | | | | | | | | |
|---|---|---|---|---|---|---|---|---|---|---|---|---|---|---|---|
| | | E1 | E2 | E3 | E4 | E5 | E6 | E7 | E8 | E9 | E10 | E11 | E12 | E13 | E14 |
| | M11 | 4 | 4 | | 4 | | 6 | | | | 4 | 6 | | 6 | |
| | M12 | | 4 | 4 | | | | 2 | | | | 2 | | | 2 |
| | M13 | | | 4 | 4 | 6 | | | 4 | 4 | | | 2 | | 2 |
| | M14 | | | | | | | | | | | 2 | 2 | 2 | 6 |

1.3uL of each solution was then spotted onto a silanized glass slide in the following configuration. Arrays with this configuration can be referred to herein as α-arrays.

```
A1    B1    C1    D1    E1
A2    B2    C2    D2    E2
A3    B3    C3    D3    E3
A4    B4    C4    D4    E4
A5    B5    C5    D5    E5
A6    B6    C6    D6    E6
A7    B7    C7    D7    E7
A8    B8    C8    D8    E8
A9    B9    C9    D9    E9
A10   B10   C10   D10   E10
A11   B11   C11   D11   E11
A12   B12   C12   D12   E12
A13   B13   C13   D13   E13
A14   B14   C14   D14   E14
```

The spotting was performed using a Biomek 2000 fluid handling robot, but other suitable methods and devices can be used. The glass slides used to create each array were laser etched with unique identifiers (IDs). After spotting the solutions, the spots were photopolymerized under a 365 nm gel-curing UV lamp (e.g., Jiadi JD818) for twenty minutes.

A total of six α-arrays were incubated with each of the 39 analytes for 10 minutes at room temperature. The arrays were then removed and air dried at room temperature for 20 minutes and further dried on a heated plate at 100°C for 20 minutes. Subsequently, the arrays were heated on a heated plate at 250°C for 5 minutes to develop their colorimetric signal profiles. The arrays were scanned with a digital scanner (e.g., EPSON Perfection v500) at 1200 dpi and saved as red, green, and blue ("RGB" or "R, G, and B") JPEG images. Each array spot was cropped using a fixed window size and then split into R, G, and B channels for further analysis. The R, G and B intensities of each spot were obtained by summing the respective R, G and B pixel intensities for each spot window.

A vector of 210 spot intensities (70 spots multiplied three color channels) was obtained from each array data point. Each vector was L1 normalized. Unsupervised hierarchical clustering analysis was performed using hierarchical clustering software "hierarchical_clustering.py" authored by Nathan Salomonis (J. David Gladstone Institutes, San Francisco California).

L2 normalization was used for both the row-wise and column-wise metrics. A heatmap was generated using the color gradient "gist_ncar." To score the clustering results, misclassified arrays were identified in a two-step process: (1) for each class of six arrays, identify the largest contiguous cluster, and (2) any array which was not part of this largest contiguous cluster was identified as misclassified. The clustering accuracy was calculated as

$$\frac{Total\ \#\ of\ arrays - \#\ of\ misclassified\ arrays}{Total\ \#\ of\ arrays} \times 100\%.$$

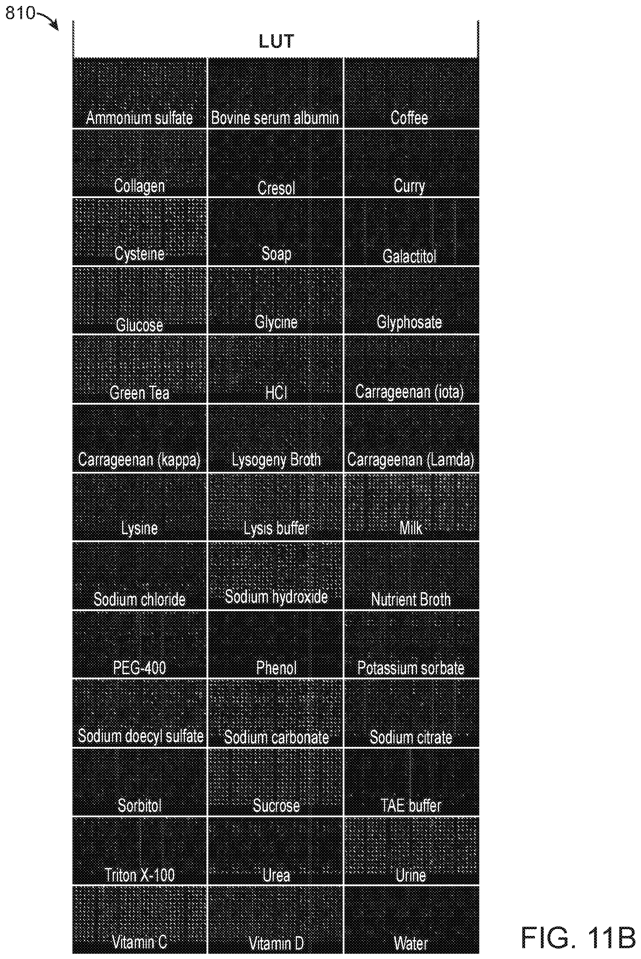

FIGS. 11A and 11B depict the scanned array images. FIG. 11A depicts the scanned array images 800 montaged using image processing software (e.g., ImageJ), showing their transmittance. FIG. 11B depicts a Look Up Table (LUT) version 810 of the arrays created by inverting the montaged version and applying the LUT "brgbcmyw.lut" in ImageJ. The chart below identifies the analyte corresponding to each box shown in FIGS. 11A and 11B, identified according to their position in FIGS. 11A and 11B.

| | | |
|---|---|---|
| Ammonium sulfate | Bovine serum albumin | Coffee |
| Collagen | Cereal | Curry |
| Cysteine | Soap | Galactitol |
| Glucose | Glycine | Glyphosate |
| Green tea | HCl | Iota Carrageenan |
| Kappa Carrageenan | Lysogeny Broth | Lambda Carrageenan |
| Lysine | Lysis buffer | Milk |
| Sodium Chloride | Sodium Hydroxide | Nutrient Broth |
| PEG400 | Phenol | Potassium sorbate |
| Sodium dodecyl sulfate (SDS) | Sodium carbonate | Sodium citrate |
| Sorbitol | Sucrose | TAE buffer |
| Triton X-100 | Urea | Urine |
| Vitamin C | Vitamin D | Distilled water |

As depicted in FIGS. 11A and 11B, each analyte exhibited a unique browning and colorimetric profile (transmittance and LUT, respectively). For example, samples such as cysteine produced more blue-shifted color profiles and samples such as glucose produced more red-shifted color profiles.

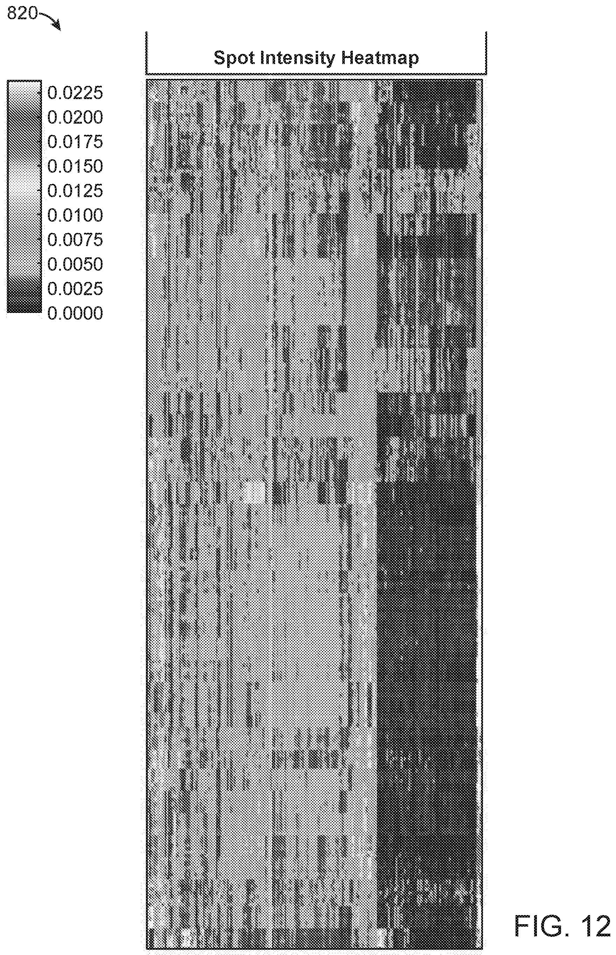

Unsupervised hierarchical clustering was performed on 234 arrays (i.e., 39 analytes with 6 arrays per analyte). Arrays and array spots were both clustered using the Euclidean distance measure. Such hierarchical clustering was performed using systems, devices, and methods described herein, e.g., as described with reference to FIGS. 9, 10, and 16. FIG. 12 depicts a spot intensity heatmap 820 of the 234 arrays (i.e., 39 analytes with 6 arrays per analyte). Each row in the heatmap 820 represents a single array for a total of 234 rows (i.e., 39 analytes each tested with 6 arrays). Each column in the heatmap 820 represents intensity for a single spot position on the arrays, split into R, G and B channels, for a total of 210 columns (i.e., 70 spots per array split into R, G, and B channels). The values of the intensity are coded according to the legend in FIG. 12, [with lower values having colors toward the bottom of the scale and higher values having colors toward the top of the scale]. The rows in the heatmap 820 are hierarchically clustered such that arrays having spot intensities that are similar to one another are placed in close proximity to one another.

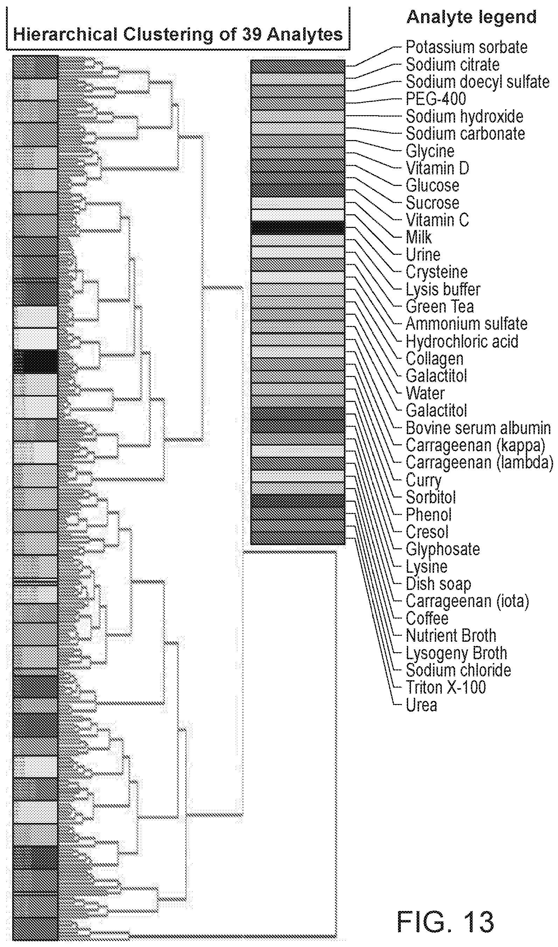

FIG. 13 depicts the output of the clustering as a dendogram 830. Each analyte is represented in the legend in order of appearance in the dendrogram 830 from top to bottom. Each row of the dendogram 830, same as the heatmap 820, represents a single array for a total of 234 rows (i.e., 39 analytes each tested with 6 arrays). As both the heatmap 820 and the dendrogram 830 include hierarchically clustered data, each row of the heatmap 820 corresponds to each respective row of the dendrogram 820. As depicted in FIG. 13, the hierarchical clustering correctly clustered 229 out of 234 arrays (i.e., 97.86% of the arrays).

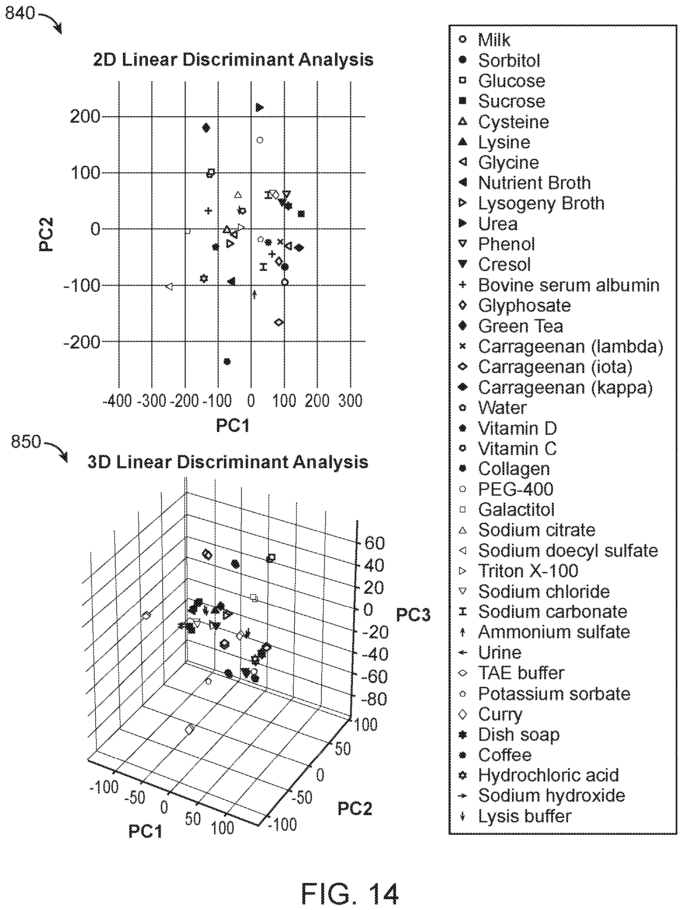

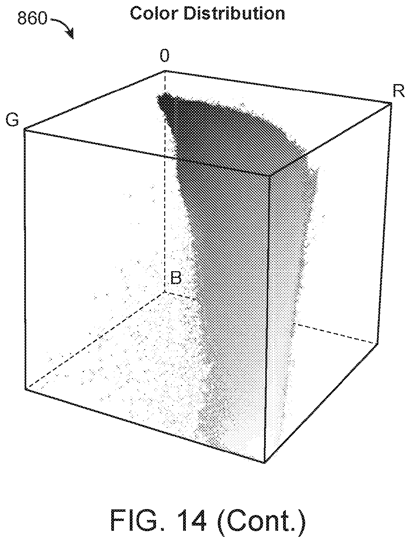

Two-dimensional (2D) and three-dimensional (3D) linear discriminant analyses were performed with the image data using the classifier "sklearn.lda.LDA" from the machine learning library "scikit-learn," version 0.16.1. The plotted output of the 2D and 3D linear discriminant analyses are depicted in graphs 840 and 850, respectively (see FIG. 14). In graphs 840 and 850, each point or symbol represents an array and corresponds to one of the analytes as identified by the legend.

Color distribution analysis was performed on the 234 arrays (i.e., 39 analytes with 6 arrays per analyte). Specifically, the general color profile of the scanned array images was obtained using the Color Inspector 3D plug-in software in ImageJ. FIG. 14 depicts the output of this color distribution analysis in graph 860. As shown, there is a bias toward red and blue colorimetric products in the data collected from the 39-analyte study. This is consistent with typical colors observed with benzil or benzoin as TLC reagents.

The five misclassified arrays included one of glucose, one or carrageenan (lambda), two of phenol, and one or lysine. Of the misclassified arrays, four were mis-clustered with other samples that had similar chemical structures and properties. In particular, one glucose array was clustered with sucrose, one carrageenan (lambda) array was clustered with carrageenan (kappa), and two phenol arrays were clustered with cresol. The remaining mis-clustered array for lysine was mistakenly clustered with sodium chloride due to a fainter signature.

This 39-analyte study demonstrates that system, devices, and methods as described herein provide an array platform that is able to profile multiple sample types with high accuracy, even when many the samples are chemically similar. For example, the lambda, kappa, and iota forms of carrageenan were clustered separately with the exception of one array. Complex mixtures, such as, for example, coffee, curry, green tea, LB media, milk, and nutrient broth, were distinguished with no errors. Different salts including, for example, potassium sorbate, sodium citrate, sodium carbonate, ammonium sulfate, and sodium chloride were also clustered with no errors. Consistent with the clustering data, the linear discriminant analysis showed that samples clustered tightly with two to three principal components (graphs 840 and 850).

### Example 2 - 29 Analyte Study

In some embodiments, systems, devices, and methods can use fluorescence or luminescence profiles to distinguish between different analytes.

α-arrays as described above (i.e., hydrogel arrays cured with DMPA) were tested with the following 29 analytes:

| Analyte | Concentration (w/v or v/v) |
|---|---|
| None | NA |
| Distilled Water | NA |
| Sodium Hydroxide | 1% |
| Sodium Citrate | 1% |
| Ammonium Sulfate | 1% |
| HCl | 1% |
| Tris Acetate EDTA (TAE) | 1X |
| Urea | 1% |
| Sucrose | 1% |
| Glucose | 1% |
| Galactitol | 1% |
| Sorbitol | 1% |
| SDS | 1% |
| Triton X-100 | 1% |
| Lysis Buffer (PBS) | 1X |
| Glycine | 1% |
| Lysine | 1% |
| Cysteine | 1% |
| Collagen | 1% |
| Glyphosate (Weed Killer) | 1% |
| Bovine Serum Albumin | 1% |
| m-Cresol | 1% |
| Lysogeny Broth (LB) | 1X |
| Nutrient Broth (NB) | 1X |
| Vitamin C | 1% |
| Green Tea Extract | 0.1% |
| Lambda Carrageenan | 0.1% |
| Coffee | 0.1% |
| Whole Milk | 10% |
| Urine | 40% |

A total of three α-arrays were incubated with each of 29 analytes for 10 minutes at room temperature, and three α-arrays were incubated with no analyte to establish a control. The arrays were then removed and air dried at room temperature for 20 minutes and further dried on a heated plate at 100°C for 20 minutes. Subsequently, the arrays were heated on a heated plate at 250°C for 5 minutes to develop their colorimetric signal profiles. Fluorescence images were captured by illuminating the arrays with UV light at 390 nm, and Apple® iPhone® night camera (version 3) software application to capture RGB JPEG images using the following manual configuration: Shutter speed: 1/15s; Auto ISO; Exposure -3.3 (range between -8.0 to +8.0).

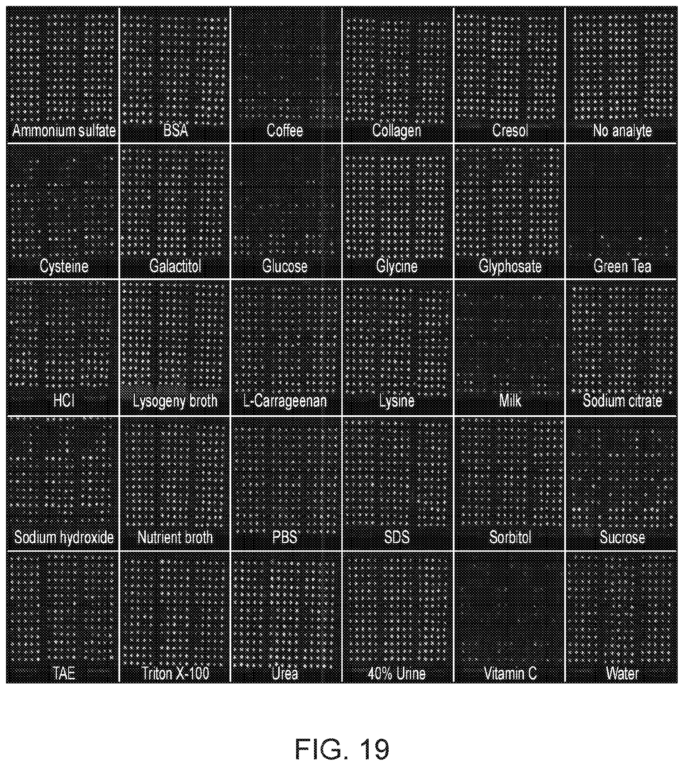

FIG. 19 depicts the fluorescence profile of the scanned array images montaged using ImageJ. As depicted, each analyte exhibits a unique fluorescence profile.

## Example 3 - Disease Classification Study

Systems, devices, and methods described herein can be used to detect multiple disease classes simultaneously without priority knowledge of analytes within tested samples. In an embodiment, hydrogel arrays as described herein can be used to detect disease classes from biofluid samples such as, for example, urine samples. The ability of such arrays to handle arbitrary sample types without or with minimal sample processing and labeling enables analysis using biofluids other than blood. The reduced sample processing required by the hydrogels also results in less analyte losses and degradation prior to analysis, which can limit detection of circulating tumor deoxyribonucleic acid (DNA) isolated from plasma.

In a study, hydrogel arrays cured with DMPA were used to detect multiple disease classes using urine samples. Urine was selected because the collection of such samples are relatively simple and non-invasive and do not require pre-processing. Urine, however, is a relatively underutilized sample type despite being informationally rich in small molecule analytes. Accordingly, systems, devices, and methods described herein can produce non-redundant information to complement other clinical lab tests as well as imaging modalities, or be used as a stand-alone test. Tests using urine can be easier to implement, more cost-effective, less invasive, and safer, and therefore can be particularly suited for disease screening or surveillance of relapse in patients.

For the study, $\alpha$-arrays as described above were used along with a second 70-spot hydrogel array, referred to as $\beta$-arrays. $\beta$-arrays include spot diversities different from the $\alpha$-arrays so as to increase sensitivity of the disease class detection.

$\beta$-arrays were created using the stock solutions F1 to F14, G1 to G9, H1 to H15, I1 to I18, J1 to J11 and K1 to K3, shown in Tables 8-12.

### Table 8

| | | Polymers F1 to F14 | | | | | | | | | | | | |
|---|---|---|---|---|---|---|---|---|---|---|---|---|---|---|
| | | F1 | F2 | F3 | F4 | F5 | F6 | F7 | F8 | F9 | F10 | F11 | F12 | F13 | F14 |
| Monomer Stock Solution (uL) | M1 | | | | | | | | | | | 5 | 5 | 5 | 9 |
| | M2 | | | | | | | | | | | 5 | | | |
| | M3 | 5 | 5 | 5 | 5 | 5 | 5 | 5 | 5 | 5 | 9 | | | | |
| | M5 | 5 | | | | | | | | | | | | 5 | |
| | M7 | | 5 | | | | | | | | | | | | 5 |
| | M8 | | | 5 | | | | | | | | | | | |
| | M9 | | | | 5 | | | | | | | | | | |
| | M10 | | | | | 5 | | | | | | | | | |
| | M11 | | | | | | 5 | | | | | | | | |
| | M12 | | | | | | | 5 | | | | | | | |
| | M13 | | | | | | | | 5 | | | | | | |
| | M14 | | | | | | | | | 5 | | | | | |
| | M15 | | | | | | | | | | 1 | | | | 1 |

**Table 9**

| | | G1 | G2 | G3 | G4 | G5 | G6 | G7 | G8 | G9 | I16 | I17 | I18 |
|---|---|---|---|---|---|---|---|---|---|---|---|---|---|
| | | \multicolumn — Polymers G1 to G9 and I16 to I18 | | | | | | | | | | | |
| Monomer Stock Solution (uL) | M5 | 5 | 5 | 5 | 5 | 5 | 5 | 5 | 5 | 9 | | | |
| | M7 | 5 | | | | | | | | | 9 | | |
| | M8 | | 5 | | | | | | | | | 9 | |
| | M9 | | | 5 | | | | | | | | | 9 |
| | M10 | | | | 5 | | | | | | | | |
| | M11 | | | | | 5 | | | | | | | |
| | M12 | | | | | | 5 | | | | | | |
| | M13 | | | | | | | 5 | | | | | |
| | M14 | | | | | | | | 5 | | | | |
| | M15 | | | | | | | | | 1 | 1 | 1 | 1 |

**Table 10**

| | | H1 | H2 | H3 | H4 | H5 | H6 | H7 | H8 | H9 | H10 | H11 | H12 | H13 | H14 | H15 |
|---|---|---|---|---|---|---|---|---|---|---|---|---|---|---|---|---|
| | | Polymers H1 to H15 | | | | | | | | | | | | | | |
| Monomer Stock Solution (uL) | M10 | 5 | 5 | 5 | 5 | | | | | | | 9 | | | | |
| | M11 | 5 | | | | 5 | 5 | 5 | | | | | 9 | | | |
| | M12 | | 5 | | | 5 | | | 5 | 5 | | | | 9 | | |
| | M13 | | | 5 | | | 5 | | 5 | | 5 | | | | 9 | |
| | M14 | | | | 5 | | | 5 | | 5 | 5 | | | | | 9 |
| | M15 | | | | | | | | | | | 1 | 1 | 1 | 1 | 1 |

**Table 11**

| | | I1 | I2 | I3 | I4 | I5 | I6 | I7 | I8 | I9 | I10 | I11 | I12 | I13 | I14 | I15 |
|---|---|---|---|---|---|---|---|---|---|---|---|---|---|---|---|---|
| | | Polymers I1 to I15 | | | | | | | | | | | | | | |
| Monomer Stock Solution (uL) | M7 | 5 | 5 | 5 | 5 | 5 | | | | | | | | | | |
| | M8 | | | | | | 5 | 5 | 5 | 5 | 5 | | | | | |
| | M9 | | | | | | | | | | | 5 | 5 | 5 | 5 | 5 |
| | M10 | 5 | | | | | 5 | | | | | 5 | | | | |
| | M11 | | 5 | | | | | 5 | | | | | 5 | | | |
| | M12 | | | 5 | | | | | 5 | | | | | 5 | | |
| | M13 | | | | 5 | | | | | 5 | | | | | 5 | |
| | M14 | | | | | 5 | | | | | 5 | | | | | 5 |

Table 12

| | | Polymers J1 to J11 and K1 to K3 | | | | | | | | | | | | | |
|---|---|---|---|---|---|---|---|---|---|---|---|---|---|---|---|
| | | J1 | J2 | J3 | J4 | J5 | J6 | J7 | J8 | J9 | J10 | J11 | K1 | K2 | K3 |
| Monomer Stock Solution (uL) | M2 | 5 | 5 | 5 | 5 | 5 | 5 | 5 | 5 | 5 | 5 | 9 | | | |
| | M3 | 5 | | | | | | | | | | | | | |
| | M5 | | 5 | | | | | | | | | | | | |
| | M7 | | | 5 | | | | | | | | | 5 | 5 | |
| | M8 | | | | 5 | | | | | | | | 5 | | 5 |
| | M9 | | | | | 5 | | | | | | | | 5 | 5 |
| | M10 | | | | | | 5 | | | | | | | | |
| | M11 | | | | | | | 5 | | | | | | | |
| | M12 | | | | | | | | 5 | | | | | | |
| | M13 | | | | | | | | | 5 | | | | | |
| | M14 | | | | | | | | | | 5 | | | | |
| | M15 | | | | | | | | | | | 1 | | | |

The β-arrays have the following configuration:

```
F1    G1    H6    I5    J1
F2    G2    H7    I6    J2
F3    G3    H8    I7    J3
F4    G4    H9    I8    J4
F5    G5    H10   I9    J5
F6    G6    H11   I10   J6
F7    G7    H12   I11   J7
F8    G8    H13   I12   J8
F9    G9    H14   I13   J9
F10   H1    H15   I14   J10
F11   H2    I1    I15   J11
F12   H3    I2    I16   K1
F13   H4    I3    I17   K2
F14   H5    I4    I18   K3
```

Urinalysis was performed on 592 study participants with the α-arrays and β-arrays. The study participants included healthy individuals (197), patients who were Hepatitis B virus-infected (93), and patients diagnosed with various stages of biopsy-proven colorectal (78), liver (144), prostate (24), and lung (56) cancer. These five diseases (Hepatitis B, colorectal cancer, liver cancer, prostate cancer, and lung cancer) were selected because they have a high prevalence in the general population and/or suffer from a lack of simple screening options despite favorable therapeutic outcomes if detected and treated early.

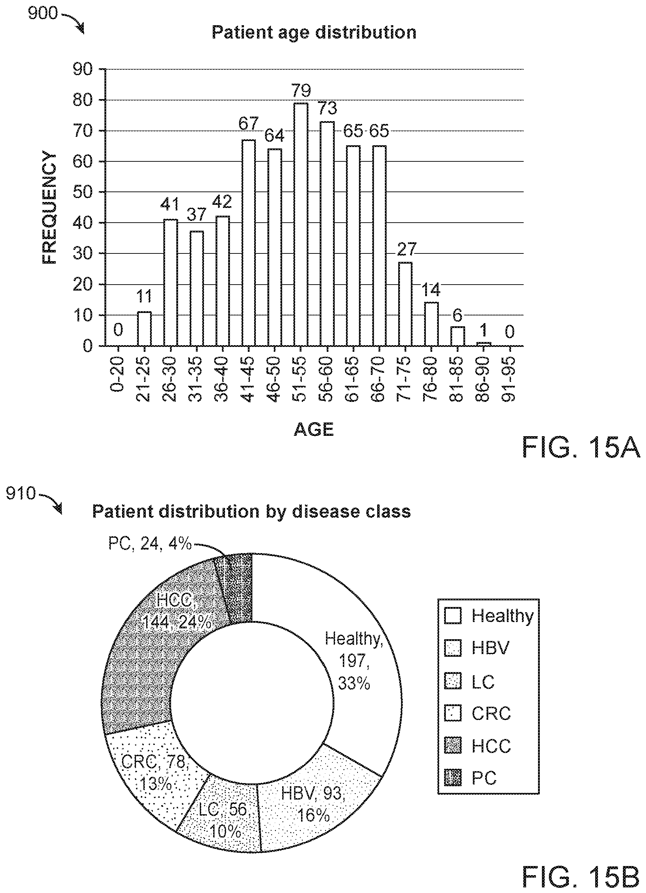

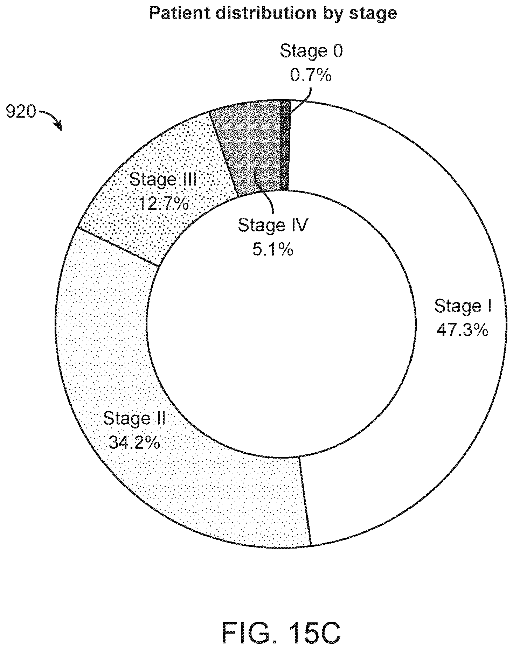

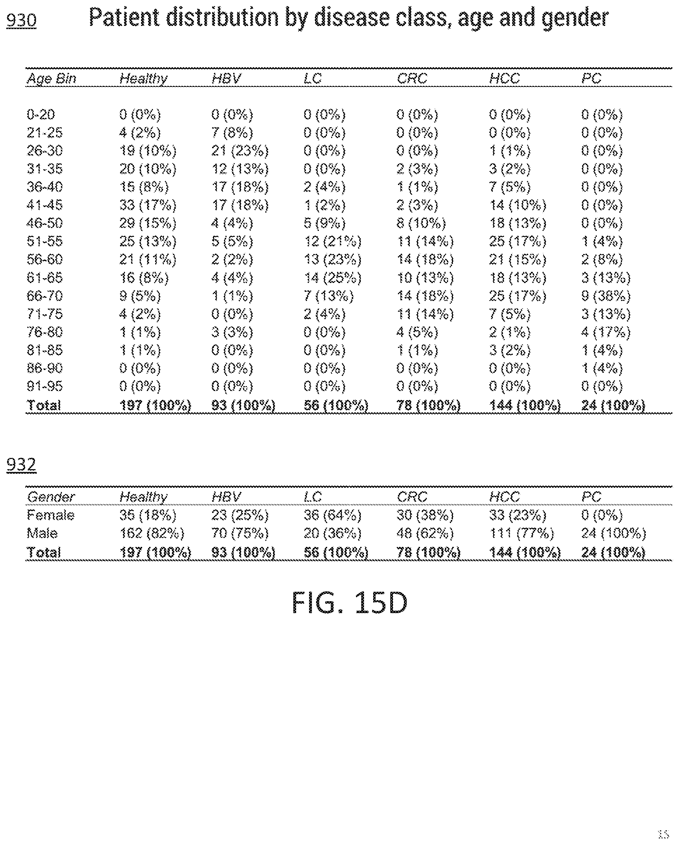

Healthy individuals had a mean age of 47.3 (with standard deviation of 12.52) and patients had a mean age of 54.4 (with standard deviation of 14.2). The demographics of the patient population are summarized in FIGS. 15A-15D. FIG. 15A depicts a bar graph 900 of the patient age distribution. FIG. 15B depicts a chart 910 of the patient distribution by disease class. FIG. 15C depicts the patient distribution by the stage of their disease using charts 920 and 922. Specifically, as depicted in FIG. 15C, 82.2% of cancer patients tested were in Stage 0 (carcinoma in situ), Stage I, or Stage II of their disease. FIG. 15D depicts the patient distribution by disease class, age, and gender in charts 930 and 932.

Sample collection was conducted according to set policies and procedures. Healthy individuals were enrolled during their routine physical examinations. Urine was collected before any clinical testing or procedures (e.g., colonoscopy) to avoid changes arising from the clinical examination itself. With some individuals, blood work, liver and renal function, tumor markers, chest x-rays and ultrasound for liver, gallbladder, pancreas, spleen and kidneys were performed.

Other healthy individuals were males greater than 40 years old, without known medical conditions, and with normal prostate ultrasound and normal cancer biomarkers. Individuals were enrolled as healthy when they did not exhibit any disease and did not have any pre-existing medical conditions (e.g., tumors, hypertension, diabetes, and kidney disease). Similar procedures were used for enrolling patients with clinically validated Hepatitis B, colorectal cancer, liver cancer, prostate cancer, and lung cancer. For these, urine was collected prior to any surgery (e.g., biopsy) and were classified under a particular disease after pathology results became available.

Each study participant sample was tested with six arrays comprising three $\alpha$-arrays and three $\beta$-arrays. Undiluted urine samples were mixed 1:1 with a fixative solution containing 50% methanol and stored at room temperature until tested. Fixed urine, 9 mL / chamber, was then added into two disposable plastic slide chambers, one containing the $\alpha$-arrays and the other containing the $\beta$-arrays. After incubation at room temperature for fifteen minutes, the arrays were removed from the chamber and excess urine removed by tapping the arrays on paper towels. The arrays were then heat cycled for about 10 minutes in an infrared oven (SMTHouse Reflow Oven T962A) with a maximum temperature of 250°C to develop their colorimetric signal profiles.

Arrays were scanned with a digital scanner (EPSON Perfection v500) at 1200 dpi and saved as RGB JPEG images. Each array spot was cropped using a fixed window size and then split into R, G and B channels for further analysis. The R, G and B intensities of each spot were obtained by summing the respective R, G and B pixel intensities for each spot window. A vector of 210 spot intensities (70 spots multiplied by three color channels) was obtained from each array data point. Each vector was L1 normalized.

The resulting vectors were used to train and validate a neural network implemented as a multilayer perceptron (MLP) with a single fully connected hidden layer. While further analysis is described herein with reference to such a classification algorithm, one of ordinary skill can appreciate that other classification algorithms can be used.

The MLP was implemented using the sklearn.neural_network.MLPClassifier model, version 0.18. The architecture of the model included 210 input nodes and a single 235-node hidden layer. A ReLU or rectified linear unit was used as the activation function, and logistic regression was performed using Limited memory Broyden-Fletcher-Goldfarb-Shanno (LBFGS) optimization algorithm. The MLP was trained and evaluated using a leave-one-out cross-validation framework, which isolated each patient sample data in turn, trained the MLP on the remaining patients, and then applied the trained MLP to the isolated patient sample to predict the class of that isolated patient sample.

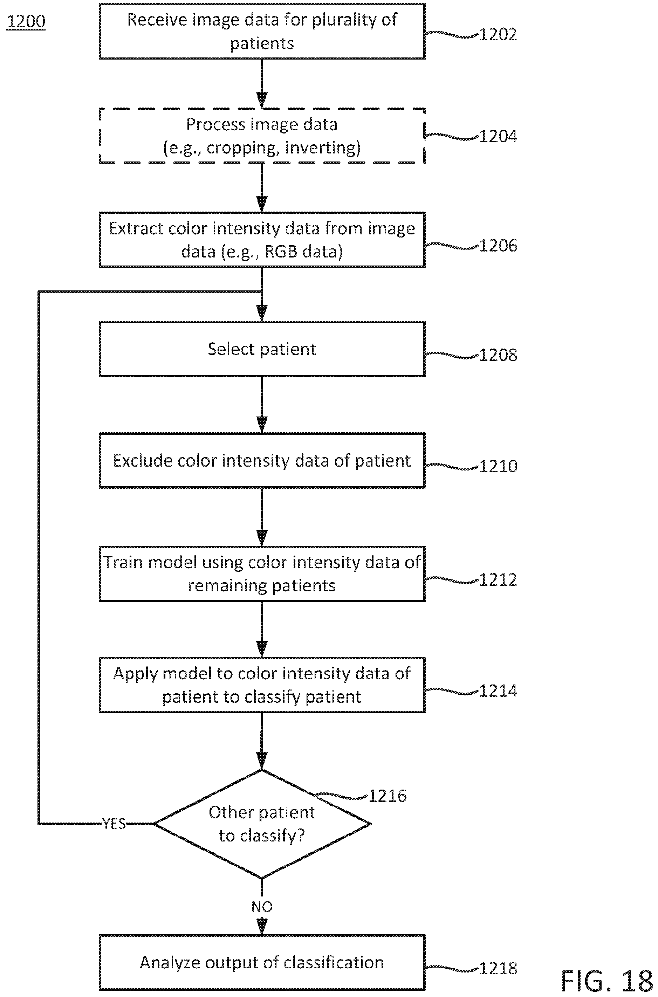

FIG. 18 provides a detailed view of an example method 1200 for training and evaluating a classification model such as, for example, the MLP model as used in the study. Image data for a plurality of patients can be received, at 1202. As described above, this image data can be image data acquired using a flatbed scanner. Optionally, the image data can be processed (e.g., cropped, inverted), at 1204. The color intensity data from the image data can be split into different color channels, e.g., R, G, and B channels and organized as a vector representative of each array data point, at 1206. In some embodiments, the vector data can be L1 normalized, as described in examples above. The leave-one-out cross-validation framework can then be implemented. Data for each patient can be selected, at 1208, and excluded, at 1210. The classification model can then be trained using data of the remaining, non-excluded patients, at 1212. The training can be supervised (e.g., including labels identifying the class of each patient) or unsupervised. The trained model can then be used to predict a disease class for the excluded patient based on that patient's color intensity data (e.g., vector), at 1214. For example, the excluded vector for that patient can be assigned a class with the highest class probability as determined using the trained model. The method 1200 can cycle through 1208-1214 until all patients have been classified (1216: NO), and then proceed to analyze the output of the classification, at 1218. This analysis can involve, for example, comparing the predicted class for each patient to the validated or known class of that patient. In some embodiments, performance statistics (e.g., sensitivity, specificity, positive predictive value and negative predictive value) can be generated based on the analysis.

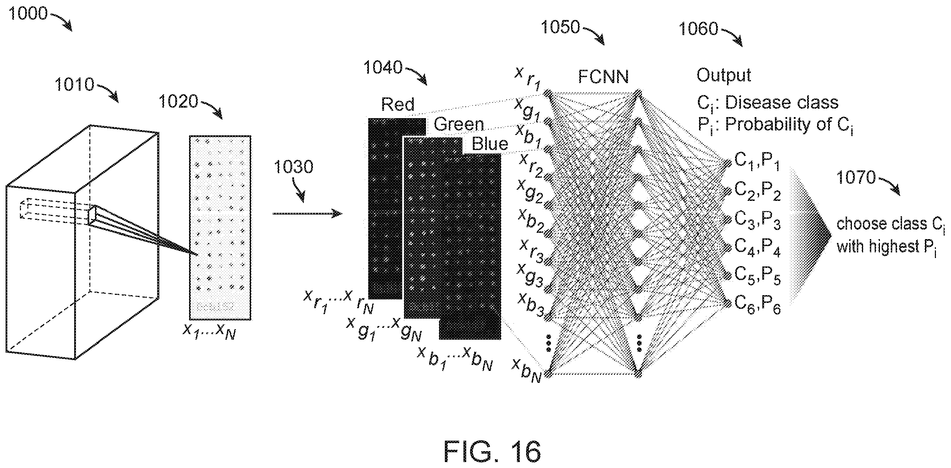

FIG. 16 depicts an example flow 1000 showing the inputs and outputs to the MLP. The flow 1000 was used in the study to evaluate the urine samples. Urine is collected from healthy, Hepatitis B, and cancer patients, at 1010. Each urine sample was used to process a predetermined number of arrays, e.g., three $\alpha$-arrays and three $\beta$-arrays for a total of six arrays, at 1020. The spots on each array can be identified as $x_1$, $x_2$, $x_3$, ... $x_n$. Vectors representing the colorimetric data captured from the arrays are then processed and separated into R, G, and B channels, at 1040. The values for each spot on the arrays corresponding to each of the channels can be represented as $x_{r1}$, $x_{r2}$, $x_{r3}$, ... $x_{rn}$ for the red channel, $x_{g1}$, $x_{g2}$, $x_{g3}$, ... $x_{gn}$ for the green channel, and $x_{g1}$, $x_{g2}$, $x_{g3}$, ... $x_{gn}$ for the green channel. One or more fully convolutional neural networks (FCNNs) can be implemented, at 1050. For example, in the instances involving three $\alpha$-arrays and three $\beta$-array, one FCNN can be trained using the data from the $\alpha$-arrays and another FCNN can be trained using the data from $\beta$-arrays. The FCNNs can output a probability ($P_i$) of each sample being of a particular disease class ($C_i$), at 1060. And the class with the highest probability for each sample can be selected as the class for that sample, at 1070.

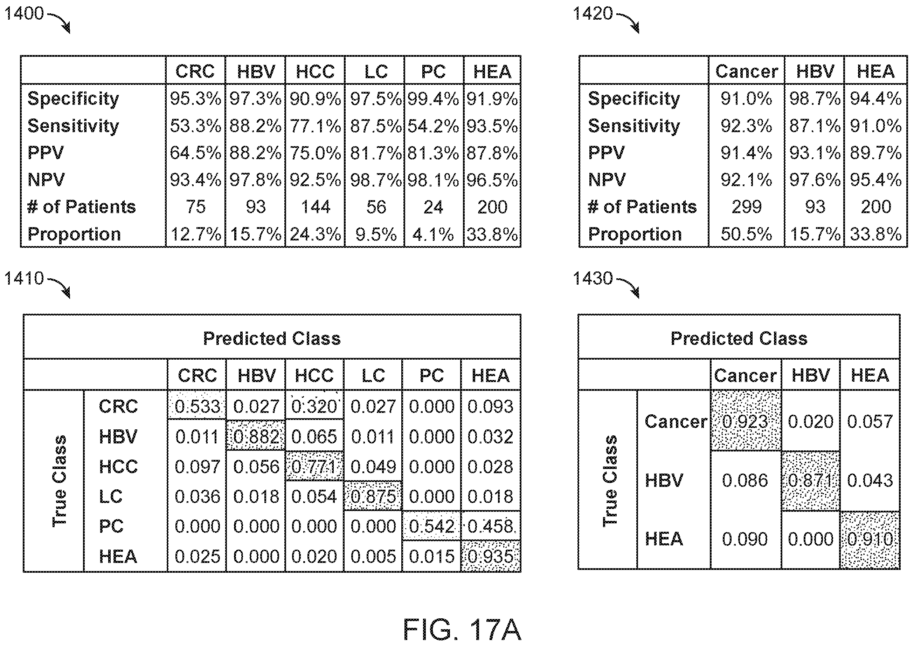

FIG. 17A depicts the performance metrics for the MLP used in the study. Three colorectal cancer patients who were in complete remission after treatment were labeled as healthy for the purposes of training and evaluating the MLP. Charts 1400 and 1410 depict the performance metrics when the MLP was trained and evaluated on six classes, i.e., healthy (HEA), Hepatitis B (HBV), colorectal cancer (CRC), liver cancer (HCC), prostate cancer (PC), and lung cancer (LC). As shown in charts 1400 and 1410, 482 out of the 592 individuals were correctly classified, providing a raw accuracy of 81.4% and normalized accuracy (i.e., the averaged accuracies of the classes) of 75.6%. Sensitivities were 53.3% for colorectal patients, 87.5% for lung cancer patients, 88.2% for HBV patients, and 93.5% for healthy individuals. With the exception of liver cancer, specificities for all other cancers were greater than 95% with prostate cancer having the highest specificity. Chart 1410 depicts the confusion matrix. In chart 1410, shaded boxes identify where an individual with a certain condition was classified as having that condition. As shown, the largest source of error was the misclassification of 45.8% prostate cancer patients as healthy. In contrast, relatively few healthy individuals (1.5%) were misclassified as having prostate cancer. The next largest two sources of error was the mutual misclassification of 32.0% colorectal cancers as liver cancer and 9.7% liver cancer as colorectal cancer. These misclassifications may have resulted from the common gastrointestinal origin of these cancers.

The analysis was repeated with colorectal, liver, lung and prostate cancer being combined into a single "cancer" class. Charts 1420 and 1430 depict the performance metrics of when evaluated with this lesser number of classes. In chart 1430, shaded boxes identify where an individual with a certain condition was classified as having that condition. As shown in charts 1420 and 1430, 539 out of 592 individuals were correctly classified, giving a raw accuracy of 91.1% and a normalized accuracy of 90.1%. In this combined "cancer" scenario, the sources of misclassification was the 9.0% healthy individuals and 8.6% HBV patients who were classified as "cancer." "Cancer" sensitivity (92.3%) and positive predictive value (91.4%) was higher than the sensitivity and positive predictive value of each cancer when evaluated as separate classes. This outcome is consistent with more cancer patients being correctly identified and explains why the normalized accuracy for the combined "cancer" analysis is 90.1% compared to 75.6% when the cancers were differentiated. The increase in "cancer" sensitivity, however, imposes a cost in increased false positives. This trend is reflected in the lower specificity (with the exception of liver cancer) and negative predictive value of the combined "cancer" analysis compared to the differentiated cancer analysis.

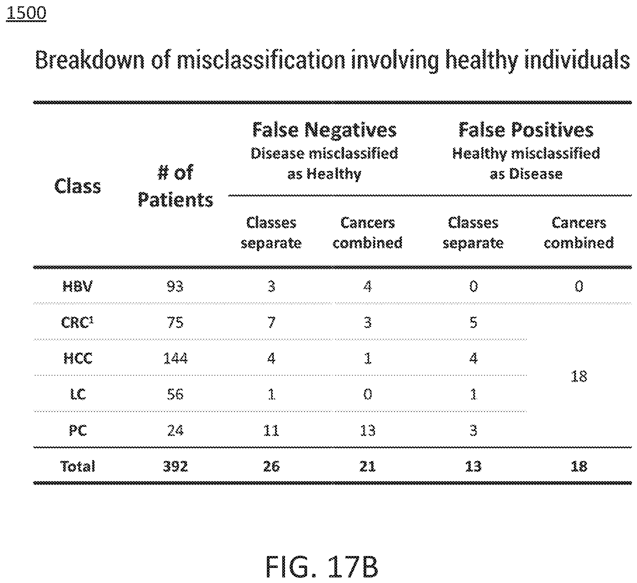

FIG. 17B depicts a chart 1500 that provides a breakdown of misclassification errors involving healthy and diseased individuals. Prostate cancer was responsible for most of the false negatives (disease misclassified as healthy) accounting for 11 out of 26 errors (42.3%) when cancers were differentiated and 13 out of 21 errors (61.9%) when cancers were combined into one "cancer" class. This may be due to prostate cancer having the lowest representation in the study participants with 24 patients. Thus, increasing this training set may improve overall classification performance. False positives (healthy misclassified as disease) by comparison were more evenly distributed over the disease classes with the exception of HBV for which there were none. There were a total of 13 false positives when cancers were differentiated and 18 when cancers were combined. There were exactly the same total number of such errors (i.e., 39) regardless of whether cancers were combined or differentiated. This indicates that the lower accuracy of the differentiated analysis is caused by increased misclassification between diseases.

The results depicted in FIGS. 17A and 17B indicate that there is sufficient differentiation between the array profiles to tell between different types of cancers. Combining cancers into one class exacts a cost in specificity, which is balanced by a gain in both sensitivity and accuracy. Both approaches (i.e., differentiated classes and combined class) are not mutually exclusive. In an embodiment, the combined class approach could be used as a sensitive primary screen followed by the differentiated approach to discern the possible tissue origin of the cancer.

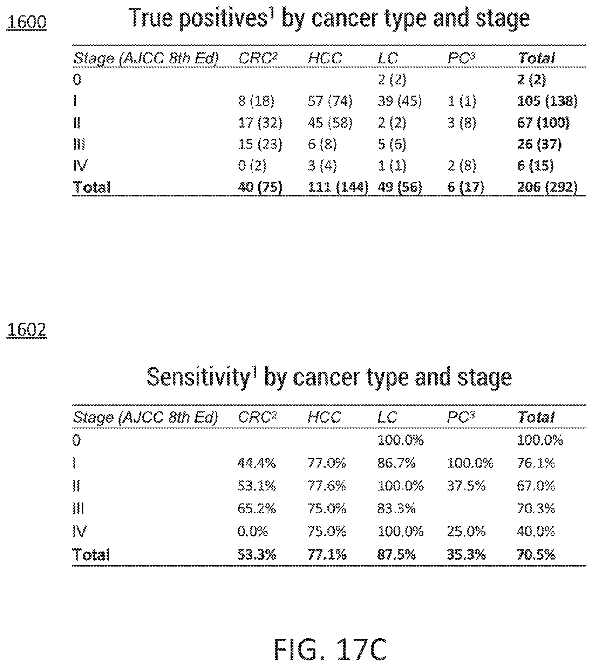

FIG. 17C includes charts 1600 and 1602 that depict the true positives and sensitivities by cancer type and stage. As shown, sensitivities were generally higher for Stages I, II and III and lower for Stage IV. This can be due to the larger representation of prostate cancer, which has lower sensitivity, within the Stage IV population.

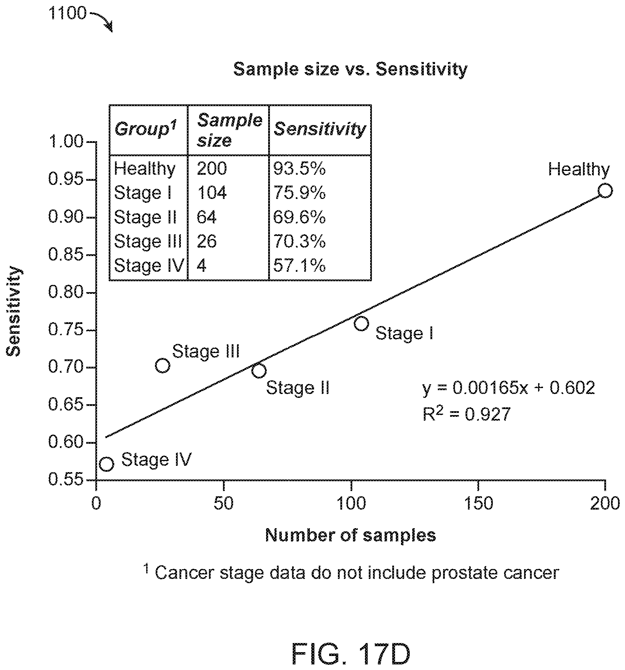

FIG. 17D depicts a graph 1100 of sensitivity based on number of samples per stage. Prostate cancer was omitted in this analysis to avoid the bias described above. Stages represented by higher sample numbers had higher sensitivity, with Stage IV (n=4) faring worst (57.1%) and Stage I (n=104) faring best (75.9%). Healthy individuals (n=200) had the highest sensitivity (93.5%). This trend suggests that there are differences in the array patterns that enable various cancer stages to be distinguished from one another. Such differences are consistent with known stepwise accretion of mutational and metabolic changes that occur during tumor development. The outcome of this data indicates that the stages of disease can be well represented in the data-driven approach described herein, e.g., because cancer at various stages can present unique signatures. Increasing data representation for different stages of cancer can therefore improve classification performance.

Unless defined otherwise, all technical and scientific terms used herein have the same meaning as commonly understood by one of ordinary skill in the art to which the invention belongs. Although any methods, compositions, reagents, cells, similar or equivalent to those described herein can be used in the practice or testing of the invention, the preferred methods and materials are described herein.

"Analytes" are entities of interest that can bind to (by covalent, ionic, physical, or any other means) and be detected on an array (such as the arrays disclosed herein). In some embodiments, analytes may include, but are not limited to, small molecules, such as vitamins and minerals, cells, proteins, peptides, nucleotides, and the like.

A "subject," as used herein, includes any animal that exhibits a symptom, or is at risk for exhibiting a symptom, which can be treated or diagnosed (e.g., with a system, device, composition or method disclosed herein or known in the art). Suitable subjects (patients) include non-human primates and, preferably, human patients, as well as laboratory animals (such as mouse, rat, rabbit, or guinea pig), farm animals, and domestic animals or pets (such as a cat or dog).

"Treatment" or "treating," as used herein, includes any desirable effect on the symptoms or pathology of a disease or condition, and may include even minimal changes or improvements in one or more measurable markers of the disease or condition being treated. "Treatment" or "treating" does not necessarily indicate complete eradication or cure of the disease or condition, or associated symptoms thereof. The subject receiving this treatment is any subject in need thereof. Exemplary markers of clinical improvement will be apparent to persons skilled in the art.

It should be emphasized that the above-described embodiments of the present disclosure are merely possible examples of implementations set forth for a clear understanding of the principles of the disclosure. Many variations and modifications may be made to the above-described embodiment(s) without departing substantially from the principles of the disclosure. All such modifications and variations are intended to be included herein within the scope of this disclosure as far as they are covered by the following claims.

Also, various concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

As used herein, the terms "about" and/or "approximately" when used in conjunction with numerical values and/or ranges generally refer to those numerical values and/or ranges near to a recited numerical value and/or range. In some instances, the terms "about" and "approximately" may mean within $\pm$ 10% of the recited value. For example, in some instances, "about 100 [units]" may mean within $\pm$ 10% of 100 (e.g., from 90 to 110). The terms "about" and "approximately" may be used interchangeably.

Some embodiments described herein relate to a computer storage product with a non-transitory computer-readable medium (also may be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also may be referred to as code or algorithm) may be those designed and constructed for the specific purpose or purposes. Examples of non-transitory computer-readable media include, but are not limited to, magnetic storage media such as hard disks, floppy disks, and magnetic tape; optical storage media such as Compact Disc/Digital Video Discs (CD/DVDs), Compact Disc-Read Only Memories (CD-ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a computer program product, which may include, for example, the instructions and/or computer code disclosed herein.

The systems, devices, and/or methods described herein may be performed by software (executed on hardware), hardware, or a combination thereof. Hardware modules may include, for example, a general-purpose processor (or microprocessor or microcontroller), a field programmable gate array (FPGA), and/or an application specific integrated circuit (ASIC). Software modules (executed on hardware) may be expressed in a variety of software languages (e.g., computer code), including C, C++, Java®, Ruby, Visual Basic®, and/or other object-oriented, procedural, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. Additional examples of computer code include, but are not limited to, control signals, encrypted code, and compressed code.

**Claims**

1. A method, comprising:

   receiving, at a processor and for a plurality of subjects, image data associated with colorimetric or luminescence signal profiles of a plurality of array spots (1) contacted with one or more samples associated with that subject and (2) heated at a predetermined temperature for a predetermined period of time, each of the plurality of array spots including a different hydrogel composition with a photoinitiator that was previously exposed to ultraviolet (UV) light;
   processing, for each of the plurality of subjects, the image data associated with that subject to produce color intensity values for each of the plurality of array spots by summing red, green, and blue pixel intensities for each of the plurality of array spots;

training, for each of the plurality of subjects, a neural network to classify that subject by calibrating the neural network using the color intensity values associated with each subject other than that subject and of the plurality of subjects, the neural network being a multilayer perceptron including an input layer having about 210 nodes, the number representing 70 spots multiplied by three color channels, and a hidden layer having between about 30 nodes to 250 nodes; and

predicting, for each of the plurality of subjects, a diagnostic class for that subject using the neural network trained for that subject.

2. The method of claim 1, wherein the neural network uses a rectifier as an activation function and performs logistic regression using a limited-memory Broyden-Fletcher-Goldfarb-Shanno (L-BFGS) algorithm.

3. The method of claim 1, wherein the diagnostic class is selected from a plurality of diagnostic classes including a healthy class and at least one cancer class.

4. The method of claim 1, wherein the diagnostic class is selected from a plurality of diagnostic classes including a healthy class, a plurality of cancer classes, and a Hepatitis B virus-infected class.

5. The method of claim 1, further comprising comparing the diagnostic class predicted for each of the plurality of subjects to a known class of each of the plurality of subjects.

**Patentansprüche**

1. Verfahren, umfassend:

Empfangen, bei einem Prozessor und für eine Vielzahl von Probanden, von Bilddaten, die kolorimetrischen oder Lumineszenzsignalprofilen einer Vielzahl von Anordnungspunkten zugeordnet sind, die (1) in Kontakt mit einer oder mehreren Proben sind, die diesem Probanden zugeordnet sind und (2) einen vorbestimmten Zeitraum lang auf eine vorbestimmte Temperatur erhitzt werden, wobei jeder der Vielzahl von Anordnungspunkten eine andere Hydrogel-Zusammensetzung mit einem Photoinitiator einschließt, der zuvor ultraviolettem (UV) Licht ausgesetzt war;

Verarbeiten der mit diesem Probanden zugeordneten Bilddaten für jeden der Vielzahl von Probanden, um Farbintensitätswerte für jeden der Vielzahl von Anordnungspunkten durch Summieren roter, grüner und blauer Pixelintensitäten für jeden der Vielzahl von Anordnungspunkten herzustellen;

Trainieren eines neuronalen Netzwerks für jeden der Vielzahl von Probanden, um diesen Probanden durch Kalibrieren des neuronalen Netzwerks unter Verwendung der Farbintensitätswerte zu klassifizieren, die jedem anderen Probanden als diesem Probanden und der Vielzahl von Probanden zugeordnet sind, wobei das neuronale Netzwerk ein mehrschichtiges Perzeptron ist, das eine Eingangsschicht, die etwa 210 Knoten aufweist, wobei die Zahl 70 Punkte multipliziert mit drei Farbkanälen darstellt, und eine verborgene Schicht einschließt, die zwischen etwa 30 Knoten und 250 Knoten aufweist; und

Vorhersagen einer Diagnoseklasse für jeden der Vielzahl von Probanden unter Verwendung des für diesen Probanden trainierten neuronalen Netzwerks.

2. Verfahren nach Anspruch 1, wobei das neuronale Netzwerk einen Gleichrichter als Aktivierungsfunktion verwendet und eine logistische Regression unter Verwendung eines Broyden-Fletcher-Goldfarb-Shanno-Algorithmus (L-BFGS) mit begrenztem Speicher durchführt.

3. Verfahren nach Anspruch 1, wobei die Diagnoseklasse aus einer Vielzahl von Diagnoseklassen ausgewählt wird, die eine Gesundheitsklasse und mindestens eine Krebsklasse einschließen.

4. Verfahren nach Anspruch 1, wobei die Diagnoseklasse aus einer Vielzahl von Diagnoseklassen ausgewählt wird, die eine Gesundheitsklasse, eine Vielzahl von Krebsklassen und eine mit dem Hepatitis-B-Virus infizierte Klasse einschließen.

5. Verfahren nach Anspruch 1, weiter umfassend Vergleichen der für jeden der Vielzahl von Probanden vorhergesagten Diagnoseklasse mit einer bekannten Klasse jedes der Vielzahl von Probanden.

**Revendications**

1. Procédé, comprenant :

   la réception, au niveau d'un processeur et pour une pluralité de sujets, de données d'image associées à des profils de signaux colorimétriques ou de luminescence d'une pluralité de points de réseau (1) mis en contact avec un ou plusieurs échantillons associés à ce sujet et (2) chauffés à une température prédéterminée pendant une période de temps prédéterminée, chacun de la pluralité de points de réseau incluant une composition d'hydrogel différente avec un photo-initiateur qui a été précédemment exposé à la lumière ultraviolette (UV) ;
   le traitement, pour chacun de la pluralité de sujets, des données d'image associées à ce sujet pour produire des valeurs d'intensité de couleur pour chacun de la pluralité de points de réseau en additionnant les intensités de pixels rouges, verts et bleus pour chacun de la pluralité de points de réseau ;
   l'entraînement, pour chacun de la pluralité de sujets, d'un réseau neuronal à classifier ce sujet en étalonnant le réseau neuronal en utilisant les valeurs d'intensité de couleur associées à chaque sujet autre que ce sujet et de la pluralité de sujets, le réseau neuronal étant un perceptron multicouche incluant une couche d'entrée présentant environ 210 nœuds, le nombre représentant 70 points multipliés par trois canaux de couleur, et une couche cachée présentant entre environ 30 nœuds et 250 nœuds ; et
   la prédiction, pour chacun de la pluralité de sujets, d'une classe de diagnostic pour ce sujet en utilisant le réseau neuronal entraîné pour ce sujet.

2. Procédé selon la revendication 1, dans lequel le réseau neuronal utilise un redresseur comme fonction d'activation et réalise une régression logistique en utilisant un algorithme Broyden-Fletcher-Goldfarb-Shanno (L-BFGS) à mémoire limitée.

3. Procédé selon la revendication 1, dans lequel la classe de diagnostic est sélectionnée parmi une pluralité de classes de diagnostic incluant une classe saine et au moins une classe de cancer.

4. Procédé selon la revendication 1, dans lequel la classe de diagnostic est sélectionnée parmi une pluralité de classes de diagnostic incluant une classe saine, une pluralité de classes de cancer et une classe infectée par le virus de l'hépatite B.

5. Procédé selon la revendication 1, comprenant en outre la comparaison de la classe de diagnostic prévue pour chacun de la pluralité de sujets à une classe connue de chacun de la pluralité de sujets.

FIG. 1

FIG. 2

300

FIG. 3

FIG. 4A

FIG. 4B

FIG. 5A

FIG. 5B

FIG. 5C

EP 4 022 284 B1

FIG. 6A

FIG. 6B

FIG. 6C

1300

**Legend**

n.s.
p≤0.05
p≤0.01
p≤0.001
p≤0.0001

**UV Time (mins)**

| | A01 | A02 | A03 | A04 | A05 | A06 | A07 | A08 | A09 | A10 | A11 | A12 | A13 | A14 |
|---|---|---|---|---|---|---|---|---|---|---|---|---|---|---|
| 20 | 8.36E-04 | 2.04E-01 | 1.56E-02 | 9.97E-01 | 7.88E-02 | 1.94E-01 | 5.54E-01 | 7.15E-01 | 3.31E-01 | 1.15E-01 | 1.39E-01 | 5.38E-02 | 1.60E-02 | 6.13E-02 |
| 60 | 1.78E-02 | 6.73E-04 | 9.50E-04 | 5.81E-01 | 3.65E-01 | 1.38E-02 | 2.68E-03 | 2.77E-02 | 2.78E-03 | 1.83E-03 | 1.22E-03 | 2.77E-02 | 4.23E-04 | 9.80E-03 |

**DMPA conc**

| | A01 | A02 | A03 | A04 | A05 | A06 | A07 | A08 | A09 | A10 | A11 | A12 | A13 | A14 |
|---|---|---|---|---|---|---|---|---|---|---|---|---|---|---|
| 1 | 0.549846 | 0.93453 | 0.123061 | 0.092608 | 0.197591 | 0.344676 | 0.937038 | 0.39354 | 0.01007 | 0.872051 | 0.000804 | 0.025025 | 0.87283 | 0.015 |
| 2 | 1.29E-02 | 2.46E-02 | 2.35E-03 | 5.46E-02 | 2.93E-03 | 2.43E-01 | 4.24E-01 | 3.75E-03 | 5.07E-03 | 3.50E-03 | 1.38E-03 | 4.43E-02 | 8.45E-01 | 2.90E-02 |
| 4 | 2.41E-02 | 6.07E-03 | 6.36E-05 | 1.77E-04 | 9.00E-04 | 8.86E-02 | 2.12E-02 | 9.75E-03 | 1.82E-04 | 5.02E-04 | 1.31E-03 | 1.18E-02 | 2.69E-02 | 2.79E-02 |

**Temp (deg C)**

| | A01 | A02 | A03 | A04 | A05 | A06 | A07 | A08 | A09 | A10 | A11 | A12 | A13 | A14 |
|---|---|---|---|---|---|---|---|---|---|---|---|---|---|---|
| 150 | 7.34E-03 | 9.45E-01 | 2.26E-01 | 4.76E-02 | 1.48E-01 | 6.65E-02 | 3.72E-01 | 2.66E-01 | 1.92E-02 | 4.62E-02 | 1.98E-01 | 1.33E-01 | 1.73E-01 | 1.87E-01 |
| 200 | 3.16E-02 | 4.70E-02 | 4.75E-02 | 2.46E-02 | 1.97E-02 | 1.05E-02 | 8.50E-01 | 3.71E-02 | 1.88E-02 | 3.43E-02 | 1.55E-03 | 4.82E-02 | 1.60E-02 | 5.78E-02 |
| 250 | 8.11E-03 | 8.85E-02 | 1.61E-02 | 8.09E-03 | 1.56E-02 | 8.73E-02 | 6.99E-02 | 2.50E-02 | 5.19E-03 | 2.16E-03 | 1.73E-03 | 6.25E-03 | 1.03E-04 | 4.71E-02 |
| 300 | 2.93E-02 | 2.35E-02 | 2.39E-02 | 1.86E-03 | 3.00E-02 | 2.63E-02 | 1.85E-01 | 9.90E-02 | 3.33E-02 | 3.66E-02 | 8.87E-02 | 6.78E-02 | 3.77E-03 | 5.88E-02 |
| 350 | 4.31E-02 | 5.32E-02 | 1.72E-02 | 1.72E-02 | 9.43E-03 | 4.12E-02 | 2.82E-02 | 1.51E-02 | 3.74E-03 | 6.81E-03 | 4.51E-03 | 2.79E-03 | 3.13E-05 | 1.12E-02 |
| 400 | 3.83E-03 | 2.08E-03 | 1.16E-04 | 4.86E-05 | 2.65E-05 | 1.26E-07 | 7.90E-04 | 1.14E-03 | 5.14E-04 | 7.25E-05 | 1.04E-03 | 8.03E-06 | 3.60E-06 | 3.38E-07 |

FIG. 7

39

**DMPA**

| Temp (deg C) | A01 | A02 | A03 | A04 | A05 | A06 | A07 | A08 | A09 | A10 | A11 | A12 | A13 | A14 |
|---|---|---|---|---|---|---|---|---|---|---|---|---|---|---|
| 150 | 5.72E-01 | 9.57E-01 | 2.44E-01 | 3.45E-01 | 4.18E-01 | 8.00E-01 | 2.05E-01 | 6.78E-01 | 7.46E-01 | 4.80E-01 | 5.68E-01 | 4.43E-01 | 4.05E-01 | 2.14E-01 |
| 200 | 1.38E-01 | 6.58E-01 | 3.01E-02 | 3.01E-01 | 4.35E-02 | 3.31E-01 | 9.50E-01 | 9.30E-01 | 3.01E-01 | 3.24E-01 | 7.00E-01 | 3.55E-01 | 1.77E-02 | 3.16E-02 |
| 250 | 2.89E-02 | 1.48E-02 | 1.80E-02 | 1.40E-01 | 2.28E-02 | 1.45E-02 | 1.60E-02 | 9.97E-02 | 1.16E-02 | 2.24E-03 | 1.22E-01 | 1.20E-02 | 1.20E-03 | 8.46E-03 |
| 300 | 7.31E-02 | 2.30E-03 | 1.46E-02 | 1.40E-01 | 1.65E-02 | 2.18E-02 | 1.01E-01 | 4.23E-02 | 1.19E-02 | 2.10E-02 | 2.98E-02 | 8.68E-02 | 5.26E-03 | 1.88E-03 |
| 350 | 4.13E-02 | 1.68E-04 | 1.79E-03 | 1.16E-02 | 1.95E-03 | 3.16E-07 | 2.42E-03 | 1.39E-03 | 5.21E-04 | 2.18E-03 | 3.55E-03 | 4.17E-03 | 1.09E-02 | 1.52E-02 |
| 400 | 8.34E-04 | 4.49E-06 | 7.44E-04 | 7.00E-04 | 2.29E-05 | 3.35E-05 | 1.61E-03 | 7.30E-04 | 9.28E-06 | 6.35E-07 | 3.10E-08 | 1.18E-08 | 6.77E-03 | 9.77E-03 |

**HCPK**

| Temp (deg C) | A01 | A02 | A03 | A04 | A05 | A06 | A07 | A08 | A09 | A10 | A11 | A12 | A13 | A14 |
|---|---|---|---|---|---|---|---|---|---|---|---|---|---|---|
| 150 | 2.00E-01 | 3.53E-01 | 1.76E-02 | 3.06E-01 | 3.31E-01 | 9.03E-02 | 4.86E-01 | 4.93E-01 | 7.24E-01 | 7.79E-01 | 5.07E-01 | 2.73E-01 | 6.46E-01 | 4.17E-01 |
| 200 | 3.62E-02 | 5.51E-03 | 1.75E-03 | 1.63E-02 | 2.35E-02 | 9.93E-04 | 9.97E-01 | 2.08E-01 | 6.88E-02 | 5.28E-02 | 4.22E-02 | 5.07E-02 | 4.37E-02 | 7.86E-03 |
| 250 | 1.21E-02 | 4.18E-03 | 4.56E-05 | 4.99E-03 | 1.58E-03 | 1.16E-04 | 1.48E-01 | 2.54E-01 | 6.12E-01 | 1.10E-02 | 4.44E-03 | 1.45E-02 | 1.73E-03 | 3.38E-02 |
| 300 | 6.25E-02 | 3.36E-03 | 8.64E-04 | 3.47E-02 | 1.25E-03 | 1.67E-03 | 7.94E-02 | 2.20E-02 | 3.14E-03 | 2.20E-03 | 3.14E-02 | 9.10E-03 | 3.10E-03 | 3.11E-02 |
| 350 | 2.88E-03 | 4.69E-03 | 6.95E-04 | 6.44E-04 | 7.96E-07 | 9.71E-07 | 3.52E-03 | 2.46E-04 | 1.63E-05 | 1.60E-04 | 2.01E-03 | 9.41E-04 | 1.80E-03 | 1.24E-02 |
| 400 | 2.30E-03 | 3.47E-04 | 1.99E-04 | 1.16E-04 | 2.48E-05 | 5.98E-07 | 6.82E-05 | 1.49E-03 | 3.08E-08 | 7.28E-08 | 1.39E-04 | 1.33E-06 | 3.35E-03 | 5.45E-03 |

**HMMP**

| Temp (deg C) | A01 | A02 | A03 | A04 | A05 | A06 | A07 | A08 | A09 | A10 | A11 | A12 | A13 | A14 |
|---|---|---|---|---|---|---|---|---|---|---|---|---|---|---|
| 150 | 1.24E-01 | 7.54E-02 | 9.12E-04 | 2.99E-03 | 7.77E-02 | 4.71E-01 | 4.79E-01 | 5.89E-01 | 1.69E-02 | 5.83E-01 | 1.62E-01 | 7.40E-01 | 9.28E-01 | 2.88E-02 |
| 200 | 2.18E-02 | 1.93E-03 | 4.32E-04 | 4.06E-03 | 6.07E-03 | 1.09E-03 | 2.16E-01 | 2.01E-01 | 5.80E-04 | 8.69E-03 | 2.71E-02 | 7.54E-02 | 1.35E-02 | 1.75E-03 |
| 250 | 5.80E-02 | 7.01E-04 | 6.95E-07 | 4.74E-03 | 6.99E-04 | 7.90E-04 | 1.87E-03 | 8.45E-04 | 1.48E-04 | 7.08E-04 | 9.29E-04 | 3.29E-04 | 6.57E-03 | 9.55E-05 |
| 300 | 2.64E-02 | 5.03E-04 | 6.20E-04 | 3.95E-04 | 1.02E-03 | 4.96E-04 | 1.85E-03 | 1.47E-03 | 3.68E-04 | 2.32E-05 | 3.67E-04 | 1.62E-03 | 8.36E-04 | 4.43E-04 |
| 350 | 1.53E-02 | 3.67E-02 | 6.88E-03 | 2.84E-05 | 6.85E-04 | 5.21E-05 | 3.98E-04 | 6.96E-07 | 3.81E-04 | 5.53E-04 | 1.66E-03 | 6.84E-04 | 4.57E-03 | 5.01E-03 |
| 400 | 8.55E-03 | 7.20E-04 | 2.03E-03 | 8.35E-04 | 4.05E-04 | 1.69E-08 | 1.96E-05 | 3.44E-04 | 4.15E-05 | 1.08E-04 | 2.08E-04 | 5.93E-08 | 3.00E-08 | 5.36E-03 |

FIG. 7(Cont.)

700

A

Contact sample to array — 702

Incubate sample on array — 704

Heat array at predefined temperature — 706

Image array — 708

B

FIG. 8

700

Prepare photoinitiator solution — 712

Prepare monomer and polymer solutions — 714

Add photoinitiator solution to each monomer and polymer solution to produce spotting solution — 716

Spot volume of each spotting solution onto surface to produce array — 718

Expose array to electromagnetic energy (e.g., UV light) — 720

A

FIG. 9

700

( B )

Receive image data from imaging an array — 722

Process image data
(e.g., cropping, inverting) — 724

Extract color intensity data from image data (e.g., RGB data) — 726

Apply trained model to color intensity data to predict diagnostic class — 728

Provide clinical recommendation based on predicted diagnostic class — 730

FIG. 10

800

| Transmittance | | |
|---|---|---|
| Ammonium sulfate | Bovine serum albumin | Coffee |
| Collagen | Cresol | Curry |
| Cysteine | Soap | Galactitol |
| Glucose | Glycine | Glyphosate |
| Green Tea | HCl | Carrageenan (iota) |
| Carrageenan (kappa) | Lysogeny Broth | Carrageenan (Lamda) |
| Lysine | Lysis buffer | Milk |
| Sodium chloride | Sodium hydroxide | Nutrient Broth |
| PEG-400 | Phenol | Potassium sorbate |
| Sodium doecyl sulfate | Sodium carbonate | Sodium citrate |
| Sorbitol | Sucrose | TAE buffer |
| Triton X-100 | Urea | Urine |
| Vitamin C | Vitamin D | Water |

FIG. 11A

810

| LUT | | |
|---|---|---|
| Ammonium sulfate | Bovine serum albumin | Coffee |
| Collagen | Cresol | Curry |
| Cysteine | Soap | Galactitol |
| Glucose | Glycine | Glyphosate |
| Green Tea | HCl | Carrageenan (iota) |
| Carrageenan (kappa) | Lysogeny Broth | Carrageenan (Lamda) |
| Lysine | Lysis buffer | Milk |
| Sodium chloride | Sodium hydroxide | Nutrient Broth |
| PEG-400 | Phenol | Potassium sorbate |
| Sodium doecyl sulfate | Sodium carbonate | Sodium citrate |
| Sorbitol | Sucrose | TAE buffer |
| Triton X-100 | Urea | Urine |
| Vitamin C | Vitamin D | Water |

FIG. 11B

820

FIG. 12

Hierarchical Clustering of 39 Analytes

Analyte legend
- Potassium sorbate
- Sodium citrate
- Sodium doecyl sulfate
- PEG-400
- Sodium hydroxide
- Sodium carbonate
- Glycine
- Vitamin D
- Glucose
- Sucrose
- Vitamin C
- Milk
- Urine
- Crysteine
- Lysis buffer
- Green Tea
- Ammonium sulfate
- Hydrochloric acid
- Collagen
- Galactitol
- Water
- Galactitol
- Bovine serum albumin
- Carrageenan (kappa)
- Carrageenan (lambda)
- Curry
- Sorbitol
- Phenol
- Cresol
- Glyphosate
- Lysine
- Dish soap
- Carrageenan (iota)
- Coffee
- Nutrient Broth
- Lysogeny Broth
- Sodium chloride
- Triton X-100
- Urea

FIG. 13

840

## 2D Linear Discriminant Analysis

850

## 3D Linear Discriminant Analysis

| | |
|---|---|
| o | Milk |
| ● | Sorbitol |
| ▫ | Glucose |
| ▨ | Sucrose |
| △ | Cysteine |
| ▲ | Lysine |
| ◁ | Glycine |
| ◀ | Nutrient Broth |
| ▷ | Lysogeny Broth |
| ▶ | Urea |
| ▽ | Phenol |
| ▼ | Cresol |
| + | Bovine serum albumin |
| ◇ | Glyphosate |
| ◆ | Green Tea |
| × | Carrageenan (lambda) |
| ◇ | Carrageenan (iota) |
| ◆ | Carrageenan (kappa) |
| o | Water |
| ● | Vitamin D |
| o | Vitamin C |
| ● | Collagen |
| o | PEG-400 |
| ▫ | Galactitol |
| △ | Sodium citrate |
| ◁ | Sodium doecyl sulfate |
| ▷ | Triton X-100 |
| ▽ | Sodium chloride |
| ⊥ | Sodium carbonate |
| ↑ | Ammonium sulfate |
| ← | Urine |
| ◇ | TAE buffer |
| o | Potassium sorbate |
| ◇ | Curry |
| ✳ | Dish soap |
| ✷ | Coffee |
| ✿ | Hydrochloric acid |
| ➤ | Sodium hydroxide |
| ↓ | Lysis buffer |

## FIG. 14

FIG. 14 (Cont.)

900

**Patient age distribution**

FIG. 15A

910

**Patient distribution by disease class**

FIG. 15B

Patient distribution by stage

FIG. 15C

<u>930</u>  Patient distribution by disease class, age and gender

| Age Bin | Healthy | HBV | LC | CRC | HCC | PC |
|---|---|---|---|---|---|---|
| 0-20 | 0 (0%) | 0 (0%) | 0 (0%) | 0 (0%) | 0 (0%) | 0 (0%) |
| 21-25 | 4 (2%) | 7 (8%) | 0 (0%) | 0 (0%) | 0 (0%) | 0 (0%) |
| 26-30 | 19 (10%) | 21 (23%) | 0 (0%) | 0 (0%) | 1 (1%) | 0 (0%) |
| 31-35 | 20 (10%) | 12 (13%) | 0 (0%) | 2 (3%) | 3 (2%) | 0 (0%) |
| 36-40 | 15 (8%) | 17 (18%) | 2 (4%) | 1 (1%) | 7 (5%) | 0 (0%) |
| 41-45 | 33 (17%) | 17 (18%) | 1 (2%) | 2 (3%) | 14 (10%) | 0 (0%) |
| 46-50 | 29 (15%) | 4 (4%) | 5 (9%) | 8 (10%) | 18 (13%) | 0 (0%) |
| 51-55 | 25 (13%) | 5 (5%) | 12 (21%) | 11 (14%) | 25 (17%) | 1 (4%) |
| 56-60 | 21 (11%) | 2 (2%) | 13 (23%) | 14 (18%) | 21 (15%) | 2 (8%) |
| 61-65 | 16 (8%) | 4 (4%) | 14 (25%) | 10 (13%) | 18 (13%) | 3 (13%) |
| 66-70 | 9 (5%) | 1 (1%) | 7 (13%) | 14 (18%) | 25 (17%) | 9 (38%) |
| 71-75 | 4 (2%) | 0 (0%) | 2 (4%) | 11 (14%) | 7 (5%) | 3 (13%) |
| 76-80 | 1 (1%) | 3 (3%) | 0 (0%) | 4 (5%) | 2 (1%) | 4 (17%) |
| 81-85 | 1 (1%) | 0 (0%) | 0 (0%) | 1 (1%) | 3 (2%) | 1 (4%) |
| 86-90 | 0 (0%) | 0 (0%) | 0 (0%) | 0 (0%) | 0 (0%) | 1 (4%) |
| 91-95 | 0 (0%) | 0 (0%) | 0 (0%) | 0 (0%) | 0 (0%) | 0 (0%) |
| Total | 197 (100%) | 93 (100%) | 56 (100%) | 78 (100%) | 144 (100%) | 24 (100%) |

<u>932</u>

| Gender | Healthy | HBV | LC | CRC | HCC | PC |
|---|---|---|---|---|---|---|
| Female | 35 (18%) | 23 (25%) | 36 (64%) | 30 (38%) | 33 (23%) | 0 (0%) |
| Male | 162 (82%) | 70 (75%) | 20 (36%) | 48 (62%) | 111 (77%) | 24 (100%) |
| Total | 197 (100%) | 93 (100%) | 56 (100%) | 78 (100%) | 144 (100%) | 24 (100%) |

# FIG. 15D

FIG. 16

1400

| | CRC | HBV | HCC | LC | PC | HEA |
|---|---|---|---|---|---|---|
| Specificity | 95.3% | 97.3% | 90.9% | 97.5% | 99.4% | 91.9% |
| Sensitivity | 53.3% | 88.2% | 77.1% | 87.5% | 54.2% | 93.5% |
| PPV | 64.5% | 88.2% | 75.0% | 81.7% | 81.3% | 87.8% |
| NPV | 93.4% | 97.8% | 92.5% | 98.7% | 98.1% | 96.5% |
| # of Patients | 75 | 93 | 144 | 56 | 24 | 200 |
| Proportion | 12.7% | 15.7% | 24.3% | 9.5% | 4.1% | 33.8% |

1420

| | Cancer | HBV | HEA |
|---|---|---|---|
| Specificity | 91.0% | 98.7% | 94.4% |
| Sensitivity | 92.3% | 87.1% | 91.0% |
| PPV | 91.4% | 93.1% | 89.7% |
| NPV | 92.1% | 97.6% | 95.4% |
| # of Patients | 299 | 93 | 200 |
| Proportion | 50.5% | 15.7% | 33.8% |

1410

| | | Predicted Class | | | | | |
|---|---|---|---|---|---|---|---|
| | | CRC | HBV | HCC | LC | PC | HEA |
| True Class | CRC | 0.533 | 0.027 | 0.320 | 0.027 | 0.000 | 0.093 |
| | HBV | 0.011 | 0.882 | 0.065 | 0.011 | 0.000 | 0.032 |
| | HCC | 0.097 | 0.056 | 0.771 | 0.049 | 0.000 | 0.028 |
| | LC | 0.036 | 0.018 | 0.054 | 0.875 | 0.000 | 0.018 |
| | PC | 0.000 | 0.000 | 0.000 | 0.000 | 0.542 | 0.458 |
| | HEA | 0.025 | 0.000 | 0.020 | 0.005 | 0.015 | 0.935 |

1430

| | | Predicted Class | | |
|---|---|---|---|---|
| | | Cancer | HBV | HEA |
| True Class | Cancer | 0.923 | 0.020 | 0.057 |
| | HBV | 0.086 | 0.871 | 0.043 |
| | HEA | 0.090 | 0.000 | 0.910 |

FIG. 17A

EP 4 022 284 B1

1500

## Breakdown of misclassification involving healthy individuals

| Class | # of Patients | False Negatives Disease misclassified as Healthy | | False Positives Healthy misclassified as Disease | |
|---|---|---|---|---|---|
| | | Classes separate | Cancers combined | Classes separate | Cancers combined |
| HBV | 93 | 3 | 4 | 0 | 0 |
| CRC[1] | 75 | 7 | 3 | 5 | |
| HCC | 144 | 4 | 1 | 4 | |
| LC | 56 | 1 | 0 | 1 | 18 |
| PC | 24 | 11 | 13 | 3 | |
| Total | 392 | 26 | 21 | 13 | 18 |

# FIG. 17B

## True positives[1] by cancer type and stage

1600

| Stage (AJCC 8th Ed) | CRC[2] | HCC | LC | PC[3] | Total |
|---|---|---|---|---|---|
| 0 | | | 2 (2) | | 2 (2) |
| I | 8 (18) | 57 (74) | 39 (45) | 1 (1) | 105 (138) |
| II | 17 (32) | 45 (58) | 2 (2) | 3 (8) | 67 (100) |
| III | 15 (23) | 6 (8) | 5 (6) | | 26 (37) |
| IV | 0 (2) | 3 (4) | 1 (1) | 2 (8) | 6 (15) |
| Total | 40 (75) | 111 (144) | 49 (56) | 6 (17) | 206 (292) |

1602

## Sensitivity[1] by cancer type and stage

| Stage (AJCC 8th Ed) | CRC[2] | HCC | LC | PC[3] | Total |
|---|---|---|---|---|---|
| 0 | | | 100.0% | | 100.0% |
| I | 44.4% | 77.0% | 86.7% | 100.0% | 76.1% |
| II | 53.1% | 77.6% | 100.0% | 37.5% | 67.0% |
| III | 65.2% | 75.0% | 83.3% | | 70.3% |
| IV | 0.0% | 75.0% | 100.0% | 25.0% | 40.0% |
| Total | 53.3% | 77.1% | 87.5% | 35.3% | 70.5% |

FIG. 17C

FIG. 17D

1200

Receive image data for plurality of patients —1202

Process image data
(e.g., cropping, inverting) —1204

Extract color intensity data from image data (e.g., RGB data) —1206

Select patient —1208

Exclude color intensity data of patient —1210

Train model using color intensity data of remaining patients —1212

Apply model to color intensity data of patient to classify patient —1214

—1216
Other patient
to classify?

YES

NO

Analyze output of classification —1218

FIG. 18

FIG. 19

Compute Device <u>1700</u>

Processor <u>1702</u>

Memory <u>1704</u>

Input/Output Device
<u>1706</u>

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62893703
- CA 2923228 A1
- US 2017175169 A1


**Non-patent literature cited in the description**

- **SAMBROOK et al.** Molecular Cloning: A Laboratory Manual. 2000
- DNA Cloning: A Practical Approach, vol. I-II
- Oligonucleotide Synthesis. 1984
- Oligonucleotide Synthesis: Methods and Applications. 2004
- Nucleic Acid Hybridization. 1985
- Nucleic Acid Hybridization: Modern Applications. 2009
- Transcription and Translation. 1984
- Animal Cell Culture. 1986
- **FRESHNEY, R.I.** Culture of Animal Cells, a Manual of Basic Technique. John Wiley & Sons, 2005
- B. Perbal, A Practical Guide to Molecular Cloning. 2010
- **FARRELL, R**. RNA Methodologies: A Laboratory Guide for Isolation and Characterization. 2005
- Poly(ethylene glycol), Chemistry and Biological Applications. ACS, 1997
- Peptide and protein PEGylation. Advanced Drug Delivery Reviews. 2002, vol. 54, 453-609
- **ZALIPSKY, S et al.** Use of functionalized Poly(Ethylene Glycols) for modification of polypeptides. *Polyethylene Glycol Chemistry: Biotechnical and Biomedical Applications*